# EUROPEAN PATENT SPECIFICATION

(11) **EP 2 240 175 B1**
(45) Date of publication and mention of the grant of the patent: **02.01.2013**
(21) Application number: 09711250.2
(22) Date of filing: 10.02.2009
(51) Int. Cl.: A61K 31/404, A61K 31/44, A61P 43/00

(54) **COMPOUNDS WITH MDR1-INVERSE ACTIVITY**
VERBINDUNGEN MIT MDR1-INVERSER AKTIVITÄT
COMPOSÉS À ACTIVITÉ MDR1 INVERSE

(30) Priority: 11.02.2008 US 27712
(43) Date of publication of application: 20.10.2010
(73) Proprietor: The Government of the United States of America as represented by the Secretary of the Department of Health and Human Services, Rockville, MD 20852-3804 (US); Institute of Enzymology, Biological Research Center, Hungarian Academy of Sciences, 1113 Budapest (HU)
(72) Inventor: HALL, Matthew, D., Washington, DC 20016 (US); GOTTESMAN, Michael, M., Bethesda, MD 20817 (US); HELLAWELL, Jennifer, L., Philadelphia, PA 19103 (US); LUDWIG, Joseph, A., Houston, TX 77005 (US); FALES, Henry, M., Silver Springs, MD 20904 (US); SALAM, Noeris, K., San Diego, CA 92122 (US); SZAKÁCS, Gergely, H-1026 Budapest (HU)
(74) Representative: Gowshall, Jonathan Vallance
(86) International application number: PCT/US2009/000861
(87) International publication number: WO 2009/102433

(56) References cited:
- WO-A-2006/009765
- KARALI N: "Synthesis and primary cytotoxicity evaluation of new 5-nitroindole-2,3-dione derivatives" EUROPEAN JOURNAL OF MEDICINAL CHEMISTRY, EDITIONS SCIENTIFIQUE ELSEVIER, PARIS, FR, vol. 37, no. 11, 1 November 2002 (2002-11-01), pages 909-918, XP004393908 ISSN: 0223-5234
- KARALI, NILGUN ET AL: "Synthesis and primary cytotoxicity evaluation of new 5-bromo-3-substituted-hydrazono-1H-2-indol inones" ARCHIV DER PHARMAZIE (WEINHEIM, GERMANY) , 335(8), 374-380 CODEN: ARPMAS; ISSN: 0365-6233, 2002, XP002555814
- KARALI ET AL: "Synthesis and structure-antituberculosis activity relationship of 1H-indole-2,3-dione derivatives" BIOORGANIC & MEDICINAL CHEMISTRY, ELSEVIER SCIENCE LTD, GB, vol. 15, no. 17, 19 July 2007 (2007-07-19), pages 5888-5904, XP022152378 ISSN: 0968-0896
- PERVEZ, H. ET AL: "Synthesis of some N4-substituted isatin-3-thiosemicarbazones" NATURAL PRODUCT RESEARCH, PART A: STRUCTURE AND SYNTHESIS , 21(13), 1178-1186 CODEN: NPRPC8, 2007, XP008114740
- OMAR, A.-MOHSEN M. E. ET AL: "Syntheses of some substituted isatin .beta.-thiosemicarbazones and isatin .beta.-hydrazonothiazoline derivatives as potential antiviral and antimicrobial agents" ARCHIV DER PHARMAZIE (WEINHEIM, GERMANY) , 317(8), 701-9 CODEN: ARPMAS; ISSN: 0365-6233, 1984, XP002555816
- GUZEL O ET AL: "Synthesis and antituberculosis activity of 5-methyl/trifluoromethoxy- 1H-indole-2,3-dione 3-thiosemicarbazone derivatives" BIOORGANIC & MEDICINAL CHEMISTRY, ELSEVIER SCIENCE LTD, GB, vol. 16, no. 19, 1 October 2008 (2008-10-01), pages 8976-8987, XP025468957 ISSN: 0968-0896 [retrieved on 2008-08-27]

## Description

### FIELD

Disclosed herein are compounds useful for the treatment of drug resistant cells.

### BACKGROUND

Multidrug resistance (MDR) conferred by the ABC transporter family that includes MDR1 (ABCB1, P-glycoprotein, P-gp), presents a significant clinical challenge for drug design and development. MDR1, for example, exhibits wide substrate specificity for structurally different drugs. This wide specificity mediates drug resistance to a variety of drugs, including Vinca alkaloids, anthracyclines, epipodophyllotoxins, taxols, actinomycin D, cardiac glycosides, immunosuppressive agents, glucocorticoids, and anti-HIV protease inhibitors. Since many drugs are substrates of MDR1, its degree of expression and functionality directly affects the therapeutic effectiveness of these agents. In particular, the multidrug resistant phenotype of malignant cells is the main obstacle in the chemotherapeutic treatment of subjects having hyperproliferative disorders. MDR1 expression is well characterized in hematological malignancies, sarcomas, and other solid cancers, and is frequently correlated with poor clinical response to chemotherapy for those tumors. Strategies employed to circumvent the reduced drug accumulation conferred by these poly-specific efflux transporters have relied heavily on the development of clinical inhibitors of MDR-1 for concurrent administration with chemotherapeutics. Although a number of these inhibitors have shown promise *in vitro,* translation to the clinic has taken longer than may have been expected, possibly due to side effects caused by inhibition of endogenous function, and alternative strategies are required.

Document WO2006/009765 discloses substituted isatin-3-thiosemicarbazones for the treatment of drug resistant cancer cells. The compound NSC73306 is the reference compound for the identification of novel compounds capable of inhibiting multidrug resistance in cancer. Other preferred compounds disclosed in this document are NSC73303, NSC73307, NSC73304, and compounds A-G which are very closely related to the presently claimed compounds.

### SUMMARY

The invention is defined in the claims.

Disclosed herein are compounds and a method for inhibiting the growth of cells in a subject by administering to the subject a compound of the formula 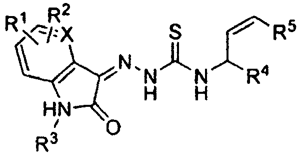 wherein R¹ and R² independently are selected from H, halogen, -OR⁶, - NR⁷R⁸, cyano, nitro, and carboxy;
X is N or CH;
R³ is H; -C(O)R⁹,-C(O)OR¹⁰ or -C(O)NR¹¹R¹²;
R⁴ is H, lower alkyl, lower alkenyl or together with R⁵ forms an optionally substituted aryl ring;
R⁵ is H, lower alkyl, lower alkenyl or together with R⁴ forms an optionally substituted aryl ring;
R⁶ is acyl, aralkyl, lower alkyl or -S(O)₂R¹³
R⁷ is acyl, aralkyl, lower alkyl or -N₂;
R⁸ is acyl, aralkyl, lower alkyl or is absent when R⁷ is -N₂;
R⁹, R¹⁰, R¹¹ and R¹² independently are selected from H, lower alkyl, aralkyl and aryl;
R¹³ is lower alkyl, aralkyl or aryl; and
when R⁴ and R⁵ form a para-methoxyphenyl moiety, at least one of R¹, R² and R³ is other than H;
provided that the compound is not one of the following compounds: 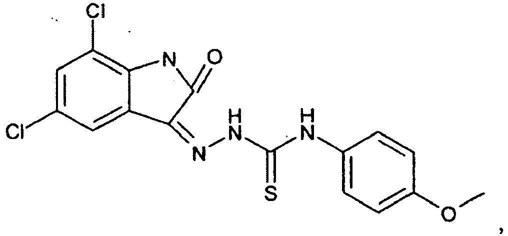 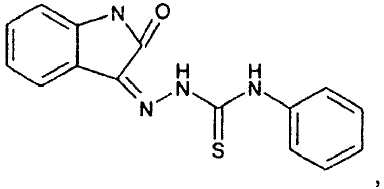 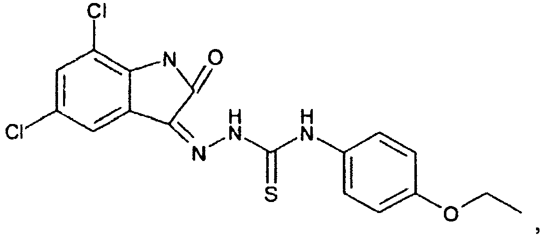 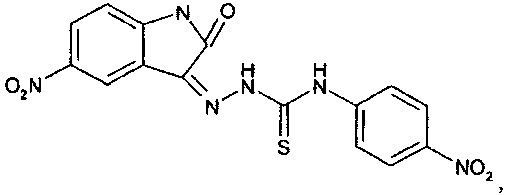 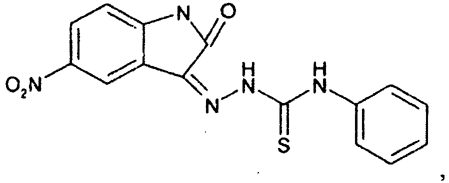 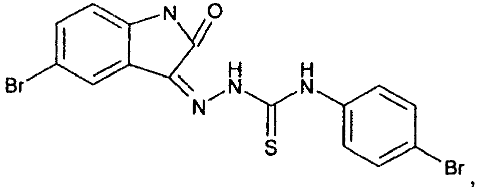 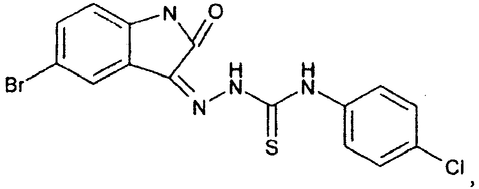 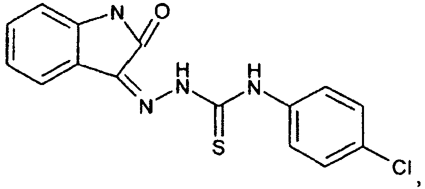 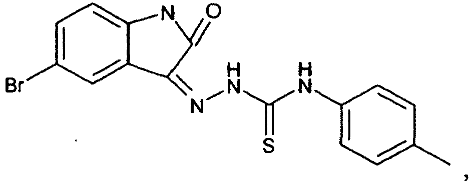 or

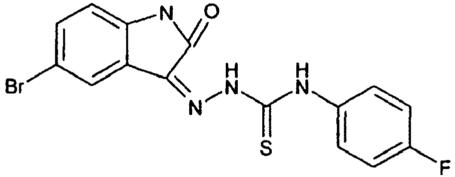

In one aspect of the invention the compounds according to the claims are particularly effective against cells that exhibit multidrug resistance. Accordingly treatment regimens employing the disclosed compounds typically involve first identifying a subject having a multidrug resistant disorder, such as a multidrug resistant tumor or infection. Certain examples of the compounds described above are not particularly cytotoxic particularly to cells that are not multidrug resistant. Moreover, in one embodiment of the invention the disclosed compounds according to the claims effectively re-sensitize multidrug resistant cells to anti-proliferative agents that are substrates for an MDR transporter. Thus, in one aspect of the invention, the disclosed compounds according to the claims are co-administered with another chemotherapeutic agent, such as an antibiotic or antineoplastic agent. In another embodiment of the invention the disclosed compounds according to the claims are both cytotoxic and render multidrug resistant cells susceptible to one or more additional chemotherapeutic agents by inhibiting an MDR transporter. Accordingly, also disclosed herein are treatment regimens and compositions formulated for combination therapy.

The foregoing and other objects, features, and advantages of the invention will become more apparent from the following detailed description, which proceeds with reference to the accompanying figures.

### BRIEF DESCRIPTION OF THE DRAWINGS

**FIG. 1** is a scatter plot and comparison of the KB-3-1 cytoxicity QSAR model applied to thirteen active thiosemicarbazones.
**FIG. 2** is a scatter plot and comparison of the KB-V1 cytoxicity QSAR model applied to twelve active thiosemicarbazones.

### DETAILED DESCRIPTION

The following explanations of terms and methods are provided to better describe the present compounds, compositions and methods, and to guide those of ordinary skill in the art in the practice of the present disclosure. It is also to be understood that the terminology used in the disclosure is for the purpose of describing particular embodiments and examples only and is not intended to be limiting.

As used herein, the singular terms "a," "an," and "the" include plural referents unless context clearly indicates otherwise. Similarly, the word "or" is intended to include "and" unless the context clearly indicates otherwise. Also, as used herein, the term "comprises" means "includes." Hence "comprising A or B" means including A, B, or A and B.

Variables such as R, R¹, R²,R³, R⁴, R⁵, R⁶, R⁷, R⁸, R⁹, R¹⁰, R¹¹, X, Y and Z, used throughout the disclosure are the same variables as previously defined unless stated to the contrary.

"Optional" or "optionally" means that the subsequently described event or circumstance can but need not occur, and that the description includes instances where said event or circumstance occurs and instances where it does not.

The term "derivative" refers to a compound or portion of a compound that is derived from or is theoretically derivable from a parent compound.

"ABC transporters" are transporter proteins belonging to the ABC protein superfamily and are capable of, in their native, active, wild type form, extruding drugs from the cells expressing them. Herein, the term "ABC transporter" also covers mutant variants of the wild type proteins retaining at least one function of the wild type, even if lacking another.

The term "acyl" refers group of the formula RC(O)- wherein R is an organic group.

The term "alkoxy" refers to a group of the formula -OR, wherein R is an organic group.

The term "alkyl" refers to a branched or unbranched saturated hydrocarbon group of I to 24 carbon atoms, such as methyl, ethyl, n-propyl, isopropyl, n-butyl, isobutyl, t-butyl, pentyl, hexyl, heptyl, octyl, decyl, tetradecyl, hexadecyl, eicosyl, tetracosyl and the like. A "lower alkyl" group is a saturated branched or unbranched hydrocarbon having from 1 to 10 carbon atoms.

The term "alkenyl" refers to a hydrocarbon group of 2 to 24 carbon atoms and structural formula containing at least one carbon-carbon double bond.

The term "alkynyl" refers to a hydrocarbon group of 2 to 24 carbon atoms and a structural formula containing at least one carbon-carbon triple bond.

The term "aliphatic" is defined as including alkyl, alkenyl, alkynyl, halogenated alkyl and cycloalkyl groups as described above. A "lower aliphatic" group is a branched or unbranched aliphatic group having from 1 to 10 carbon atoms.

The term "amine" or "amino" refers to a group of the formula -NRR', where R and R' can be, independently, hydrogen or an alkyl, alkenyl, alkynyl, aryl, aralkyl, cycloalkyl, halogenated alkyl, or heterocycloalkyl group described herein.

The term "amide group" is represented by the formula -C(O)NRR', where R and R' independently can be a hydrogen, alkyl, alkenyl, alkynyl, aryl, aralkyl, cycloalkyl, halogenated alkyl, or heterocycloalkyl group described herein. Carboxyl" refers to a -COOH radical. Substituted carboxyl refers to -COOR where R is aliphatic, heteroaliphatic, alkyl, heteroalkyl, or a carboxylic acid or ester.

The term "aryl" refers to any carbon-based aromatic group including, but not limited to, benzene, naphthalene, etc. The term "aromatic" also includes "heteroaryl group," which is defined as an aromatic group that has at least one heteroatom incorporated within the ring of the aromatic group. Examples of heteroatoms include, but are not limited to, nitrogen, oxygen, sulfur, and phosphorous. The aryl group can be substituted with one or more groups including, but not limited to, alkyl, alkynyl, alkenyl, aryl, halide, nitro, amino, ester, ketone, aldehyde, hydroxy, carboxylic acid, or alkoxy, or the aryl group can be unsubstituted. The term "alkyl amino" refers to alkyl groups as defined above where at least one hydrogen atom is replaced with an amino group.

The term "hydroxyl" is represented by the formula -OH. The term "alkoxy group" is represented by the formula -OR, where R can be an alkyl group, optionally substituted with an alkenyl, alkynyl, aryl, aralkyl, cycloalkyl, halogenated alkyl, or heterocycloalkyl group as described above.

The terms "halogenated alkyl" or "haloalkyl group" refer to an alkyl group as defined above with one or more hydrogen atoms present on these groups substituted with a halogen (F, Cl, Br, I).

The term "cycloalkyl" refers to a non-aromatic carbon-based ring composed of at least three carbon atoms. Examples of cycloalkyl groups include, but are not limited to, cyclopropyl, cyclobutyl, cyclopentyl, cyclohexyl, and the like. The term "heterocycloalkyl group" is a cycloalkyl group as defined above where at least one of the carbon atoms of the ring is substituted with a heteroatom such as, but not limited to, nitrogen, oxygen, sulfur, or phosphorous.

"Carbonyl" refers to a radical of the formula -C(O)-. Carbonyl-containing groups include any substituent containing a carbon-oxygen double bond (C=O), including acyl groups, amides, carboxy groups, esters, ureas, carbamates, carbonates and ketones and aldehydes, such as substituents based on -COR or -RCHO where R is an aliphatic, heteroaliphatic, alkyl, heteroalkyl, hydroxyl, or a secondary, tertiary, or quaternary amine.

"Carboxyl" refers to a -COOH radical. Substituted carboxyl refers to -COOR where R is aliphatic, heteroaliphatic, alkyl, heteroalkyl, or a carboxylic acid or ester.

Multidrug resistance (MDR) refers to the ability of target cells and microorganisms, particularly cancer cells and mycobacterial cells, to resist the effects of different -often structurally and functionally unrelated- cytotoxic compounds. MDR can develop after sequential or simultaneous exposure to various drugs. MDR also can develop before exposure to many compounds to which a cell or microorganism may be found to be resistant. Multidrug resistance is discussed in greater detail in Kuzmich et al., "Detoxification Mechanisms and Tumor Cell Resistance to Anticancer Drugs," particularly section VII, "The Multidrug-Resistant Phenotype (MDR)," Medical Research Reviews, 1991, 11, 185-217, particularly 208-213; and in Georges et al., "Multidrug Resistance and Chemosensitization: Therapeutic Implications for Cancer Chemotherapy," Advances in Pharmacology, 1990, 21, 185-220.

Although MDR may be caused by a variety of factors, most commonly MDR is associated with overexpression of P-glycoprotein (P-gp). P-gp is a member of a superfamily of membrane proteins, termed adenosine triphosphate (ATP)-binding cassette (ABC) proteins, which behave as ATP-dependent transporters and/or ion channels for a wide variety of substrates. P-gp is a multiple transmembrane-spanning glycoprotein. Transfection experiments with the P-gp gene (MDR1, or ABCB1) have demonstrated that P-gp confers MDR upon drug-sensitive tumor cells by providing an energy-dependent efflux pump that lowers the intracellular concentration of the cytotoxic agent, thereby allowing survival of the cell.

The term "neoplasm" refers to an abnormal cellular proliferation, which includes benign and malignant tumors, as well as other proliferative disorders.

The term "subject" includes both human and veterinary subjects.

"Transport protein" refers to a protein that acts to remove chemotherapeutic substances from cells. Examples of transport proteins include, without limitation, P-glycoprotein, the protein product of the MDR1 gene. Expression of such transport proteins confers resistance to numerous chemotherapeutic agents and sometimes entire classes of chemotherapeutics, including Vinca alkaloids, anthracyclines, epipodophyllotoxins, actinomycin D and taxanes. P-glycoprotein is over-expressed in certain chemotherapy resistant tumors and is upregulated during disease progression following chemotherapy in other malignancies. MRP, another ABC family transporter, confers a multidrug resistance phenotype that includes many natural product drugs, but is distinct from the resistance phenotype associated with P-gp. In addition to P-gp and MRP there may be other transporters that are involved in cytotoxic drug resistance. In the case of natural product drugs, resistant cell lines have been described that display a multidrug resistant phenotype associated with a drug accumulation deficit, but do not overexpress P-gp or MRP.

"Treatment" refers to a therapeutic intervention that ameliorates a sign or symptom of a disease or pathological condition after it has begun to develop. As used herein, the term "ameliorating," with reference to a disease or pathological condition, refers to any observable beneficial effect of the treatment. The beneficial effect can be evidenced, for example, by a delayed onset of clinical symptoms of the disease in a susceptible subject, a reduction in severity of some or all clinical symptoms of the disease, a slower progression of the disease, an improvement in the overall health or well-being of the subject, or by other parameters well known in the art that are specific to the particular disease. The phrase "treating a disease" refers to inhibiting the full development of a disease or condition, for example, in a subject who is at risk for a disease such as cancer, particularly a metastatic cancer. By the term "coadminister" is meant that each of at least two compounds be administered during a time frame wherein the respective periods of biological activity overlap. Thus, the term includes sequential as well as coextensive administration of two or more drug compounds. "Treating multidrug resistance" means increasing or restoring sensitivity of multidrug resistant cells to therapeutic agents. Treating multidrug resistance also may include inhibiting the development of multidrug resistance in nonresistant cells.

The term "prodrug" also is intended to include any covalently bonded carriers that release a disclosed compound or a parent thereof *in vivo* when the prodrug is administered to a subject. Since prodrugs often have enhanced properties relative to the active agent pharmaceutical, such as, solubility and bioavailability, the compounds disclosed herein can be delivered in prodrug form. Thus, also contemplated are prodrugs of the presently claimed compounds, methods of delivering prodrugs and compositions containing such prodrugs. Prodrugs of the disclosed compounds typically are prepared by modifying one or more functional groups present in the compound in such a way that the modifications are cleaved, either in routine manipulation or *in vivo,* to yield the parent compound. In particular, ester prodrugs are specifically contemplated herein. Similarly, prodrugs include compounds having an amino or sulfhydryl group functionalized with any group that is cleaved to yield the corresponding free amino or free sulfhydryl group. Examples of prodrugs include, without limitation, compounds having a hydroxy, amino and/or sulfhydryl group acylated with an acetate, formate, or benzoate group.

Protected derivatives of the disclosed compound also are contemplated. The term "protecting group" or "blocking group" refers to any group that when bound to a functional group prevents or diminishes the group's susceptibility to reaction. "Protecting group" generally refers to groups well known in the art which are used to prevent selected reactive groups, such as carboxy, amino, hydroxy, mercapto and the like, from undergoing undesired reactions, such as nucleophilic, electrophilic, oxidation, reduction and the like. The terms "deprotecting," "deprotected," or "deprotect," as used herein, are meant to refer to the process of removing a protecting group from a compound.

It is understood that substituents and substitution patterns of the compounds described herein can be selected by one of ordinary skill in the art to provide compounds that are chemically stable and that can be readily synthesized by techniques known in the art and further by the methods set forth in this disclosure. Reference will now be made in detail to the present preferred embodiments.

### I. Compounds having MDR-inverse activity

Disclosed herein are drug compounds that have MDR-inverse activity and thus are effective against multidrug-resistant cells. Examples of the disclosed compounds have been found to have, *inter alia,* efficacy in directly treating multidrug resistant cells, rendering multidrug resistant cells susceptible to other chemotherapeutics and in some instances reversing multidrug resistance.

Exemplary compounds of the invention according to the claims disclosed herein have the structure: 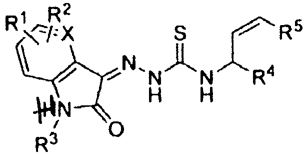 wherein R¹ and R² independently are selected from H, fluoro and iodo
X is CH;
R³ is H
R⁴ is H or together with R⁵ forms an optionally substituted phenyl or naphthyl ring;
R⁵ is H, or together with R⁴ forms an optionally substituted phenyl or naphthyl ring;
and
when R⁴ and R⁵ form a *para*-methoxyphenyl moiety, at least one of R¹, or R² is other than H;
provided that the compound is not one of the following compounds: 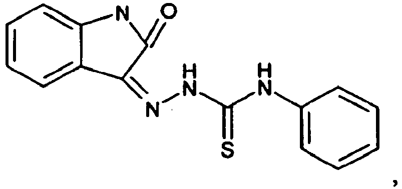 or

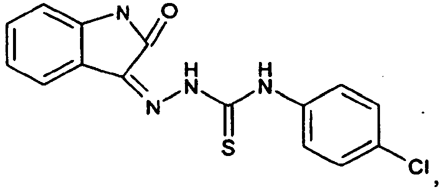

In particular examples of the invention the disclosed MDR-inverse compounds are represented by the formula 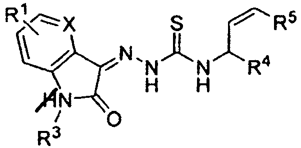 wherein R¹, R³, R⁴, R⁵ and X are as set forth above. In particular disclosures, the substructure 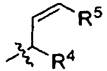 represents an optionally substituted aryl group, wherein typically the optional substitutions are electron-withdrawing groups. Certain examples of such substructures can be represented by 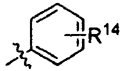 wherein R¹⁴ represents halogen, haloalkyl, such as trifluoromethyl, -OR¹⁵ wherein R¹⁵ is an acyl group, cyano, nitro or carboxy.

Other compounds disclosed herein having MDR-inverse activity include those of the formula 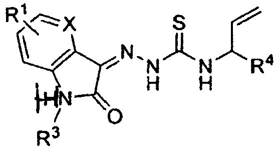 wherein X, R¹, R³ and R⁴ are as set forth above; or the formula 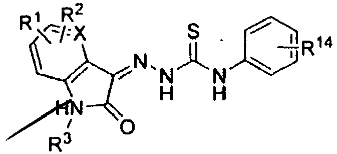 wherein R¹⁴ is H, halogen, -OR¹⁵, -NR¹⁶R¹⁷, cyano, nitro or carboxy;
R¹⁵ is acyl, aralkyl or lower alkyl;
R¹⁶ is acyl, aralkyl, lower alkyl or -N₂; and
R¹⁷ is acyl, aralkyl, lower alkyl or is absent when R¹⁶ is -N₂.

With reference to the formula above, R¹⁴ may be an *ortho, meta* or *para* substituent on the phenyl ring. Such compounds have the formulas: 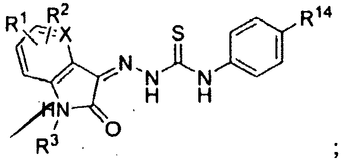 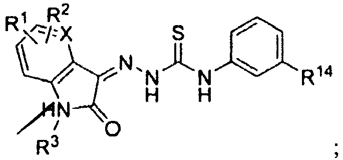 or

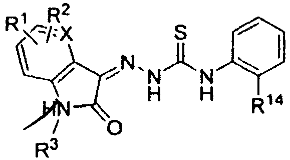

In certain examples of the disclosure R¹⁴ is -OR¹⁵ and R¹⁵ is a haloalkyl group, such as trifluoromethyl, difluoromethyl, pentafluoroethyl .

In certain examples of the disclosure, the compound may have the formula 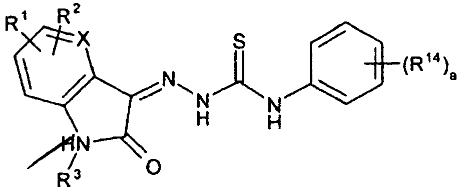 wherein a is 0 to 5;
R¹⁴ is halogen, -OR¹⁵, -NR¹⁶R¹⁷, cyano, nitro, haloalkyl, lower alkyl or carboxy, and each R¹⁴ may be the same or different;
R¹⁵ is acyl, aralkyl or lower alkyl;
R¹⁶ is acyl, aralkyl, lower alkyl or -N₂; and
R¹⁷ is acyl, aralkyl, lower alkyl or is absent when R¹⁶ is -N₂.

In certain examples of the disclosure R¹⁴ is fluoro or fluoroalkyl (e.g., trifluoromethyl, difluoromethyl, pentafluoroethyl or the like).

In certain examples of the disclosure R¹⁴ is fluoro, fluoroalkyl, methyl, or nitro; X is CH; and R¹, R² and R³ are each H.

In certain examples of the disclosure there is an R¹⁴ group at the *para* position on the phenyl ring.

In certain examples of the disclosure, subscript a is 2 to 5 meaning that there are at least two A R¹⁴ groups on the phenyl ring.

In certain examples of the disclosure of the formulas above, X is N, thus forming a heteroaromatic ring.

In one example of the disclosed compounds R¹ is a halogen, and more particularly certain disclosed compounds are represented by the formula 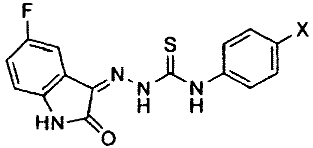 wherein X is an electron withdrawing group. In other disclosures X is an electron donating group. Examples of both electron withdrawing and electron donating groups may be lone pair donating groups as well, such as an amino, halo, phenol or alkoxy group. For example, halo groups, such as bromo substituents are electron withdrawing groups but also can function as lone pair donating groups by delocalizing electron density to the attached phenyl ring. In particular examples X is selected from H, -OCH₃, -CH₃, -CH₂CH₃, -F, -Cl, -Br, -I, -CF₃, -OCF₃, -NO₂, phenyl, -N₃, -CN, -OH, -NH₂, -NMe₂, -COOH and -SO₃⁻. In certain examples X is a hydrogen bond acceptor. Hydrogen bond-accepting groups are well known to those of skill in the art, but typically include groups having a lone pair of electrons to engage in hydrogen bonding. Examples of such groups include, without limitation, alkoxy, acyl and amino groups.

Particular examples of the disclosed MDR-inverse compounds include : 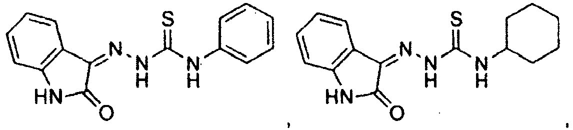 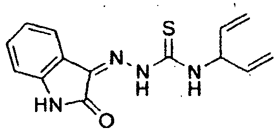 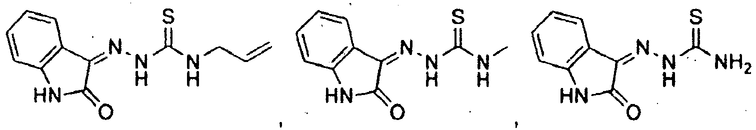 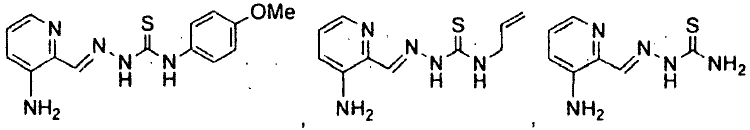 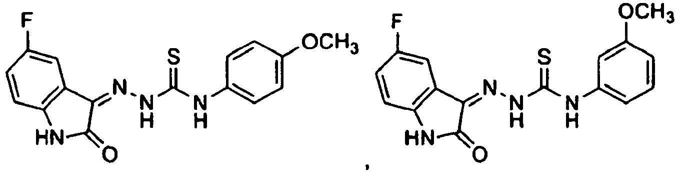 or

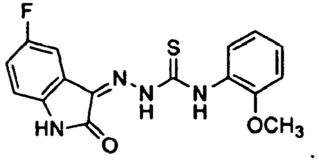

### II. Methods for Synthesis

Exemplary methods for making the disclosed thiosemicarbazone compounds are disclosed herein and additional methods for making the compounds will be apparent to those of skill in the art of organic synthesis upon consideration of the present specification.

One approach to the synthesis of the disclosed thiosemicarbazones involves the reaction of hydrazine or a protected derivative thereof with a phenylisothiocyanate, followed by the condensation of the resultant thiosemicarbazide with an isatin derivative, to produce the desired thiosemicarbazone, such as an isatin-β-thiosemicarbazone. A general method for preparing the disclosed compounds is illustrated by Scheme 1: 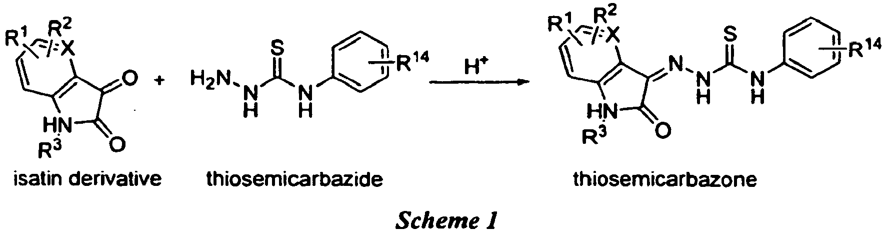 With reference to Scheme 1, equimolar amounts of isatin and thiosemicarbazide are dissolved in a protic solvent, such as ethanol, optionally with the addition of an acid catalyst, such as a few drops of acetic acid to initiate the reaction.

In particular examples, the phenylthiosemicarbazide was synthesized by the reaction of hydrazine with a substituted phenylisothiocyanate of choice, such as p-methoxyphenylisothiocyanate. For example and with reference to Scheme 2, the reaction of hydrazine with *p*-methoxyphenylisothiocyanate yields 4-(4-methoxyphenyl)-3-thiosemicarbazide.

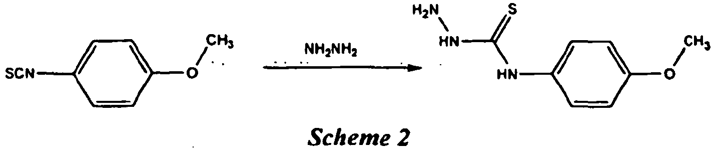

As illustrated in Scheme 3, 4-(4-methoxyphenyl)-3-thiosemicarbazide is then condensed with isatin to produce the corresponding thiosemicarbazone.

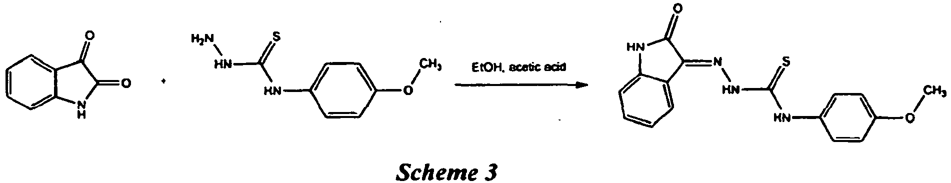

In certain examples the reaction between hydrazine and *p*-halogen phenylisothiocyanates, such as *para*-fluorophenylisothiocyanate, resulted in an insoluble precipitate that was unsuitable for further condensation with an isatin derivative. It was determined that the precipitate was a 2:1 combination of thiocyanate and hydrazine (mw = 338 gmol⁻¹) that formed even when hydrazine was added drop-wise to the excess thiocyanate (Scheme 4). 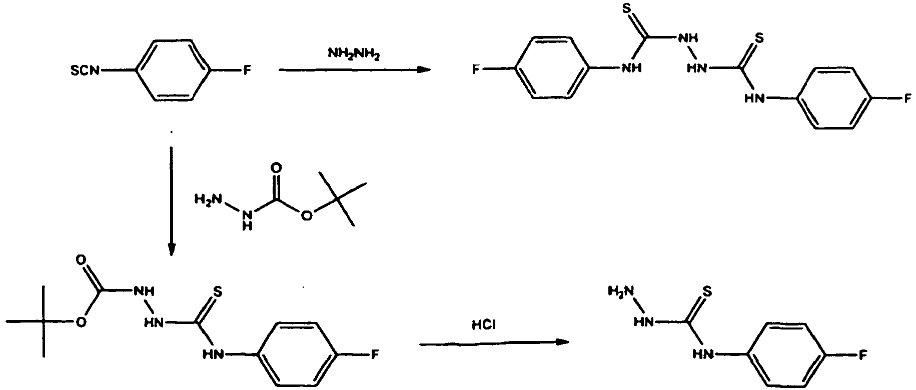 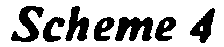 In this instance, a *t*-Boc protected form of hydrazine, *t*-butylcarbazate, which is effectively monofunctional hydrazine, the *t*-Boc protected thiosemicarbazide could be produced, from which the thiosemicarbazone was recovered under acidic conditions.

### III. Pharmaceutical Compositions and Methods for their Use

Another aspect of the disclosure includes pharmaceutical compositions prepared for administration to a subject and which include a therapeutically effective amount of one or more of the currently disclosed compounds. The therapeutically effective amount of a disclosed compound will depend on the route of administration, the species of subject and the physical characteristics of the subject being treated. Specific factors that can be taken into account include disease severity and stage, weight, diet and concurrent medications. The relationship of these factors to determining a therapeutically effective amount of the disclosed compounds is understood by those of skill in the art.

Pharmaceutical compositions for administration to a subject can include carriers, thickeners, diluents, buffers, preservatives, surface active agents and the like in addition to the molecule of choice. Pharmaceutical compositions can also include one or more additional active ingredients such as antimicrobial agents, antiinflammatory agents, anesthetics, and the like. Pharmaceutical formulations can include additional components, such as carriers. The pharmaceutically acceptable carriers useful for these formulations are conventional. Remington's Pharmaceutical Sciences, by E. W. Martin, Mack Publishing Co., Easton, PA, 19th Edition (1995), describes compositions and formulations suitable for pharmaceutical delivery of the compounds herein disclosed.

In general, the nature of the carrier will depend on the particular mode of administration being employed. For instance, parenteral formulations usually contain injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

Pharmaceutical compositions disclosed herein include those formed from pharmaceutically acceptable salts and/or solvates of the disclosed compounds. Pharmaceutically acceptable salts include those derived from pharmaceutically acceptable inorganic or organic bases and acids. Particular disclosed compounds possess at least one basic group that can form acid-base salts with acids. Examples of basic groups include, but are not limited to, amino and imino groups. Examples of inorganic acids that can form salts with such basic groups include, but are not limited to, mineral acids such as hydrochloric acid, hydrobromic acid, sulfuric acid or phosphoric acid. Basic groups also can form salts with organic carboxylic acids, sulfonic acids, sulfo acids or phospho acids or N-substituted sulfamic acid, for example acetic acid, propionic acid, glycolic acid, succinic acid, maleic acid, hydroxymaleic acid, methylmaleic acid, fumaric acid, malic acid, tartaric acid, gluconic acid, glucaric acid, glucuronic acid, citric acid, benzoic acid, cinnamic acid, mandelic acid, salicylic acid, 4-aminosalicylic acid, 2-phenoxybenzoic acid, 2-acetoxybenzoic acid, embonic acid, nicotinic acid or isonicotinic acid, and, in addition, with amino acids, for example with α-amino acids, and also with methanesulfonic acid, ethahesulfonic acid, 2-hydroxymethanesulfonic acid, ethane-1,2-disulfonic acid, benzenedisulfonic acid, 4-methylbenzenesulfonic acid, naphthalene-2- sulfonic acid, 2- or 3-phosphoglycerate, glucose-6-phosphate or *N-*cyclohexylsulfamic acid (with formation of the cyclamates) or with other acidic organic compounds, such as ascorbic acid. In particular, suitable salts include those derived from alkali metals such as potassium and sodium, alkaline earth metals such as calcium and magnesium, among numerous other acids well known in the pharmaceutical art.

Certain compounds include at least one acidic group that can form an acid-base salts with an inorganic or organic base. Examples of salts formed from inorganic bases include salts of the presently disclosed compounds with alkali metals such as potassium and sodium, alkaline earth metals, including calcium and magnesium and the like. Similarly, salts of acidic compounds with an organic base, such as an amine (as used herein terms that refer to amines should be understood to include their conjugate acids unless the context clearly indicates that the free amine is intended) are contemplated, including salts formed with basic amino acids, aliphatic amines, heterocyclic amines, aromatic amines, pyridines, guanidines and amidines. Of the aliphatic amines, the acyclic aliphatic amines, and cyclic and acyclic di- and tri- alkyl amines are particularly suitable for use in the disclosed compounds. In addition, quaternary ammonium counterions also can be used.

Particular examples of suitable amine bases (and their corresponding ammonium ions) for use in the present compounds include, without limitation, pyridine, *N*,*N*-dimethylaminopyridine, diazabicyclononane, diazabicycloundecene, *N*-methyl-*N*-ethylamine, diethylamine, triethylamine, diisopropylethylamine, mono-, bis- or tris- (2-hydroxyethyl)amine, 2-hydroxy-*tert*-butylamine, tris(hydroxymethyl)methylamine, *N*,*N*-dimethyl-*N*-(2- hydroxyethyl)amine, tri-(2-hydroxyethyl)amine and *N*-methyl-D-gtucamine. For additional examples of "pharmacologically acceptable salts," see Berge et al., J. Pharm. Sci. 66:1 (1977).

Compounds disclosed herein can be crystallized and can be provided in a single crystalline form or as a combination of different crystal polymorphs. As such, the compounds can be provided in one or more physical form, such as different crystal forms, crystalline, liquid crystalline or non-crystalline (amorphous) forms. Such different physical forms of the compounds can be prepared using, for example different solvents or different mixtures of solvents for recrystallization. Alternatively or additionally, different polymorphs can be prepared, for example, by performing recrystallizations at different temperatures and/or by altering cooling rates during recrystallization. The presence of polymorphs can be determined by X-ray crystallography, or in some cases by another spectroscopic technique, such as solid phase NMR spectroscopy, IR spectroscopy, or by differential scanning calorimetry.

In one embodiment, the presently disclosed compounds are useful for the treatment of hyperproliferative disorders wherein the hyperproliferative cells exhibit MDR or are likely to develop MDR. In general, MDR is likely to develop in proliferative disorders being treated with an MDR-inducing chemotherapeutic agent. Chemotherapeutics that tend to induce MDR are known to those of skill in the arts of pharmacology and oncology and include, for example Vinca alkaloids, anthracyclines, epipodophyllotoxins, taxols, actinomycin D, cardiac glycosides, immunosuppressive agents, glucocorticoids, and anti-HIV protease inhibitors.

One aspect of the present disclosure includes methods for treating a hyperproliferative disorder by administering a therapeutically effective amount of the disclosed compounds to a subject in need thereof. For example, particular proliferative disorders that can be so treated include solid tumors, such as sarcomas and carcinomas, include fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, lymphoid malignancy, pancreatic cancer, breast cancer, lung cancers, ovarian cancer, prostate cancer, hepatocellular carcinoma, squamous cell carcinoma, basal cell carcinoma, adenocarcinoma, sweat gland carcinoma, sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, bladder carcinoma, and CNS tumors (such as a glioma, astrocytoma, medulloblastoma, craniopharyogioma, ependymoma, pinealoma, hemangioblastoma, acoustic neuroma, oligodendroglioma, menangioma, melanoma, neuroblastoma and retinoblastoma).

The present disclosure also provides methods to treat hyperproliferative disorders that are characterized multidrug resistance. By way of example, the presently disclosed compounds and compositions can be used to inhibit multidrug resistant prostate, breast, colon, bladder, cervical, skin, testicular, kidney, ovarian, stomach, brain, liver, pancreatic or esophageal cancer, or lymphoma, leukemia or multiple myeloma. In certain embodiments the disclosed compounds and compositions are used to treat a subject is at risk of developing a metastatic proliferative disorder.

Examples of hematological tumors that can be treated as disclosed herein include leukemias, including acute leukemias (such as acute lymphocytic leukemia, acute myelocytic leukemia, acute myelogenous leukemia and myeloblastic, promyelocytic, myelomonocytic, monocytic and erythroleukemia), chronic leukemias (such as chronic myelocytic (granulocytic) leukemia, chronic myelogenous leukemia, and chronic lymphocytic leukemia), polycythemia vera, lymphoma, Hodgkin's disease, non-Hodgkin's lymphoma (indolent and high grade forms), multiple myeloma, Waldenstrom's macroglobulinemia, heavy chain disease, myelodysplastic syndrome, hairy cell leukemia and myelodysplasia.

The therapeutically effective amount of the compound or compounds administered can vary depending upon the desired effects and the factors noted above. Typically, dosages will be between about 0.01 mg/kg and 250 mg/kg of the subject's body weight, and more typically between about 0.05 mg/kg and 100 mg/kg, such as from about 0.2 to about 80 mg/kg, from about 5 to about 40 mg/kg or from about 10 to about 30 mg/kg of the subject's body weight. Thus, unit dosage forms can be formulated based upon the suitable ranges recited above and a subject's body weight. The term "unit dosage form" as used herein refers to a physically discrete unit of therapeutic agent appropriate for the subject to be treated.

Alternatively, dosages are calculated based on body surface area and from about 1 mg/m² to about 200 mg/m², such as from about 5 mg/m² to about 100 mg/m² will be administered to the subject per day. In particular embodiments, administration of the therapeutically effective amount of the compound or compounds involves administering to the subject from about 5 mg/m² to about 50 mg/m², such as from about 10 mg/m² to about 40 mg/m² per day. It is currently believed that a single dosage of the compound or compounds is suitable, however a therapeutically effective dosage can be supplied over an extended period of time or in multiple doses per day. Thus, unit dosage forms also can be calculated using a subject's body surface area based on the suitable ranges recited above and the desired dosing schedule.

It is contemplated that in some embodiments the disclosed compounds are used in combination with other types of treatments, such as cancer treatments. For example the disclosed inhibitors may be used with other chemotherapies, including those employing an anti-proliferative agent, such as, without limitation, microtubule binding agent, a toxin, a DNA intercalator or cross-linker, a DNA synthesis inhibitor, a DNA and/or RNA transcription inhibitor, an enzyme inhibitor, a gene regulator, enediyne antibiotics and/or an angiogenesis inhibitor. In one embodiment the presently disclosed compounds are used to render a neoplasm susceptible to one or more anti-proliferative compounds to which it is resistant. Additionally, the disclosed compounds can be used in combination with radiation therapy, surgery, or other modalities of cancer therapy.

"Microtubule binding agent" refers to an agent that interacts with tubulin to stabilize or destabilize microtubule formation thereby inhibiting cell division. Examples of microtubule binding agents that can be used in conjunction with the presently disclosed compounds include, without limitation, paclitaxel, docetaxel, vinblastine, vindesine, vinorelbine (navelbine), the epothilones, colchicine, dolastatin 15, nocodazole, podophyllotoxin and rhizoxin. Analogs and derivatives of such compounds also can be used and will be known to those of ordinary skill in the art. For example, suitable epothilones and epothilone analogs for incorporation into the present compounds are described in International Publication No. WO 2004/018478, which is incorporated herein by reference. Taxoids, such as paclitaxel and docetaxel are currently believed to be particularly useful as therapeutic agents in combination with the presently disclosed compounds. Examples of additional useful taxoids, including analogs of paclitaxel are taught by U.S. Patent Nos. 6,610,860 to Holton, 5,530,020 to Gurram et al. and 5,912,264 to Wittman et al. Each of these patents is incorporated herein by reference.

Suitable DNA and/or RNA transcription regulators for use with the disclosed compounds include, without limitation, actinomycin D, daunorubicin, doxorubicin and derivatives and analogs thereof also are suitable for use in combination with the presently disclosed compounds.

DNA intercalators, cross-linking agents and alkylating agents that can be used in combination therapy with the disclosed compounds include, without limitation, cisplatin, carboplatin, oxaliplatin, mitomycins, such as mitomycin C, bleomycin, chlorambucil, cyclophosphamide, isophosphoramide mustard and derivatives and analogs thereof.

DNA synthesis inhibitors suitable for use as therapeutic agents include, without limitation, methotrexate, 5-fluoro-5'-deoxyuridine, 5-fluorouracil and analogs thereof.

Examples of suitable enzyme inhibitors for use in combination with the presently disclosed compounds include, without limitation, camptothecin, etoposide, formestane, trichostatin and derivatives and analogs thereof.

Suitable therapeutics for use with the presently disclosed compounds that affect gene regulation include agents that result in increased or decreased expression of one or more genes, such as, without limitation, raloxifene, 5-azacytidine, 5-aza-2'-deoxycytidine, tamoxifen, 4-hydroxytamoxifen, mifepristone and derivatives and analogs thereof.

The term "angiogenesis inhibitor" is used herein, to mean a molecule including, but not limited to, biomolecules, such as peptides, proteins, enzymes, polysaccharides, oligonucleotides, DNA, RNA, recombinant vectors, and small molecules that function to inhibit blood vessel growth. Angiogenesis inhibitors are known in the art and examples of suitable angiogenesis inhibitors include, without limitation, angiostatin K1-3, staurosporine, genistein, fumagillin, medroxyprogesterone, SFTI-1, suramin, interferon-alpha, metalloproteinase inhibitors, platelet factor 4, somatostatin, thromobospondin, endostatin, thalidomide, and derivatives and analogs thereof.

Other therapeutic agents, particularly anti-tumor agents, that may or may not fall under one or more of the classifications above, also are suitable for administration in combination with the presently disclosed compounds. By way of example, such agents include adriamycin, apigenin, erlotinib, gefitinib, temozolomide, rapamycin, topotecan, carmustine, melphalan, mitoxantrone, irinotecanetoposide, tenoposide, zebularine, cimetidine, and derivatives and analogs thereof.

Suitable dosages and treatment regimes for administering the above-identified therapeutic agents are known to those of ordinary skill in the art of oncology and also are described, for example, in Physicians' Cancer Chemotherapy Drug Manual 2005 By Edward Chu and Vincent T. DeVita (ISBN 0763734616), which is incorporated herein by reference. Such dosages and treatment regimens can be used in combination with a presently disclosed MDR-inverse compound

The compounds disclosed herein may be administered orally, topically, transdermally, parenterally, via inhalation or spray and may be administered in dosage unit formulations containing conventional non-toxic pharmaceutically acceptable carriers, adjuvants and vehicles.

Typically, oral administration or administration via implantation or intravenously, such as via injection is preferred. However the particular mode of administration employed may be dependent upon the particular disease, condition of patient, toxicity of compound and other factors as will be recognized by a person of ordinary skill in the art.

Preferred therapeutic agents are identified herein by assessing their *in vitro* activity in a cytotoxicity assay. In this assay, certain disclosed therapeutic agents exhibit *in vitro* IC₅₀ values against a model cell line of less than about 20 µM, such as less than about 10 µM, such as from about 0.1 nM to about 1 µM, in particular from about 1 nM or 5 nM to about 500 nM, such as from about 50 nM to about 200 nM.

Suitable cell lines against which the disclosed compounds may be assessed are well known to those of skill in the art and include, by way of example, 4T1 breast cancer cells. Active compounds also can be identified and evaluated using MDR cell lines.

The observed, selective cytotoxicity of the described above was primarily assessed with respect to two biological properties: (1) The absolute cytotoxicity (measured using the MTT assay) of compounds against parental P-gp-negative KB-3-1 adenocarcinoma cells; and (2) MDR1 selectivity, as indicated by greater sensitivity in KB-V1 cells compared to KB-3-1 cells. Exemplary compounds were also tested against the KB-V1 adenocarcinoma cell line that expresses high levels of P-gp (the protein product of MDR1). The KB-V1 cell line was originally developed by step-wise selection of KB-3-1 cells in the drug vinblastine (*see,* Shen et al. Multiple drug-resistant human KB carcinoma cells independently selected for high-level resistance to colchicine, adriamycin, or vinblastine show changes in expression of specific proteins. J Biol Chem 1986, 261, 7762-70, which is incorporated herein by reference). MDR1 selectivity is determined by the ratio of IC₅₀ against KB-3-1 cells divided by its IC₅₀ against KB-V1 cells. A value > 1 indicates that the compound kills P-gp expressing cells more effectively than parental cells, resulting in so-called MDR1-inverse activity (*see,* Ludwig et al. Selective toxicity of NSC73306 in MDR1-positive cells as a new strategy to circumvent multidrug resistance in cancer. Cancer Res 2006, 66, 4808-15). Alternatively, a value < 1 indicates that the P-gp expressing cells are resistant to the compound, relative to parental cells, as is normally observed for drugs effluxed by P-gp (Gottesman, M. M. Mechanisms of cancer drug resistance. Annu Rev Med 2002, 53, 615-27). The cytotoxicity of several exemplary compounds disclosed herein is summarized in Tables 1 and 2.

The compounds according to present invention are indicated with an asterisk (*).

**Table 1**

| **Compound** | **IC₅₀, KB 3-1 (µM)** | **IC₅₀, KB-V1 (µM)** | **MDR1 selectivity^{a}** |
|---|---|---|---|
| 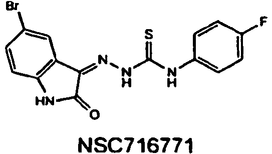 | 13.1 ± 4.8 | 5.8 ± 2.5 | 2.3 |
| 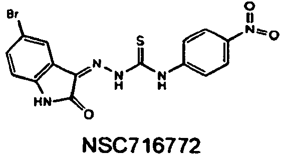 | 28.4 ± 5.2 | 4.0 ± 0.6 | 7.1 |
| 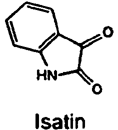 | >50 | >50 | - |
| 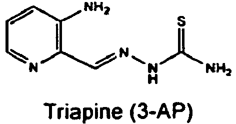 | 1.4 ± 2.5 | 5.9 ± 2.6 | 0.2 |
| 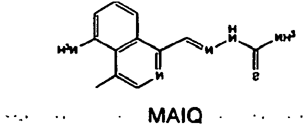 | 1.9 ± 0.8 | 2.1 ± 0.3 | 0.9 |
| 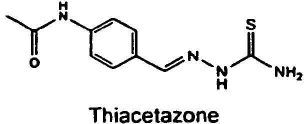 | >50 | >50 | - |
| 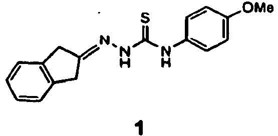 | >50 | >50 | - |
| 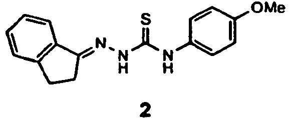 | >50 | >50 | - |
| 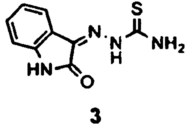 | >50 | >50 | - |
| 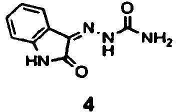 | >50 | >50 | - |
| 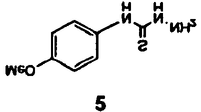 | >50 | >50 | - |
| 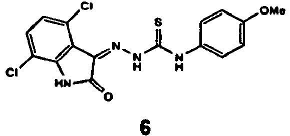 | n/a | n/a | n/a |
| 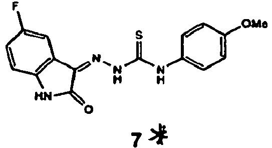 | 39.3 ± 23.3 | 5.2 ± 1.1 | 7.5 |
| 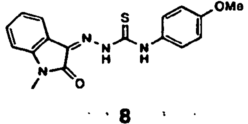 | 19.6 ± 1.7 | 9.3 ± 2.8 | 2.1 |
| 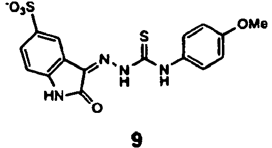 | >50 | >50 | - |
| 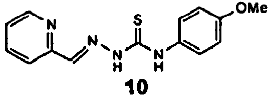 | 3.4 ± 1.5 | 2.4 ± 0.5 | 1.4 |
| 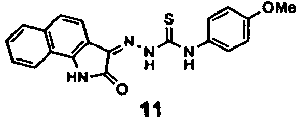 | 4.4 ± 3.6 | 30.3 ± 5.9 | 0.1 |
| 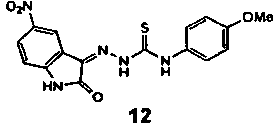 | 26.3 ± 5.5 | 6.7 ± 2.9 | 4.0 |
| 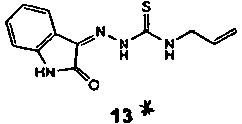 | 26.1 ± 2.6 | 20.7 ± 7.7 | 1.3 |
| 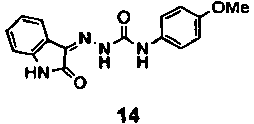 | 27.7 ± 3.3 | >50 | - |
| 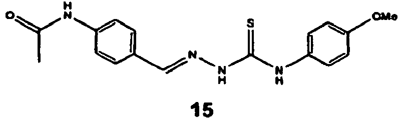 | >50 | >50 | - |

**Table 2**

| | | | | | | |
|---|---|---|---|---|---|---|
| | 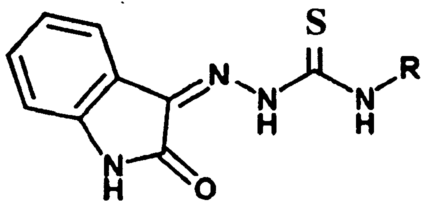 | | | | | |

| **Compound** | **R group** | **IC₅₀, KB-3-1** | **SD** | **IC₅₀, KB-V1** | **SD** | **MDR1 select.** |
|---|---|---|---|---|---|---|
| M16* | 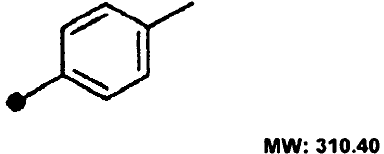 | 24.00 | 3.51 | 2.60 | 0.22 | 9.23 |
| M17* | 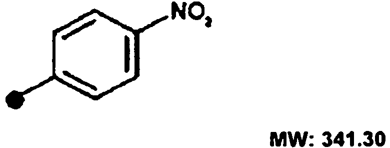 | 17.15 | 4.16 | 2.07 | 0.07 | 8.27 |
| M14* | 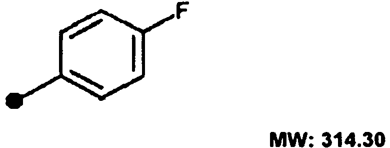 | 14.15 | 1.89 | 1.92 | 0.10 | 7.36 |
| M13* | 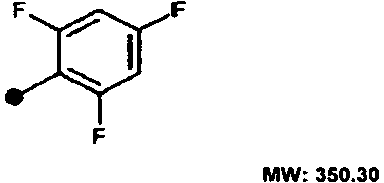 | 38.37 | 1.89 | 7.28 | 1.07 | 5.27 |
| NSC73306 | 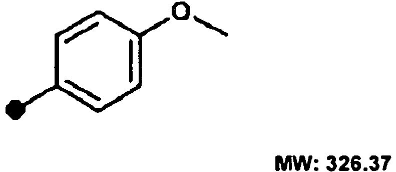 | 14.2 | 0.2 | 3.3 | 1.3 | 4.3 |
| M9* | 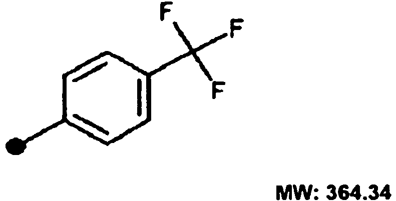 | 8.59 | 1.28 | 2.10 | 0.54 | 4:09 |
| M10* | 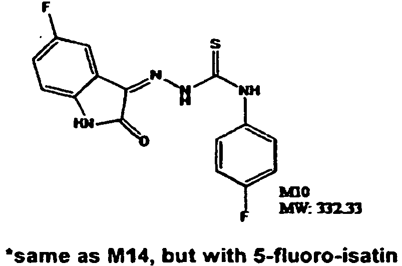 | 3.72 | 0.57 | 0.91 | 0.50 | 4.07 |
| M15 | 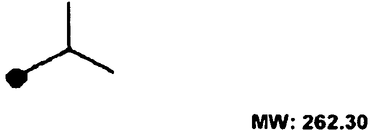 | 39.37 | 0.51 | 10.25 | 0.28 | 3.84 |
| M21 | 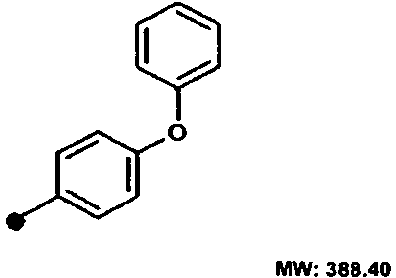 | 5.72 | 0.18 | 1.67 | 0.42 | 3.44 |
| M23* | 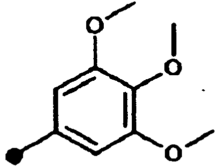 | 11.09 | 0.42 | 3.49 | 0.61 | 3.18 |
| | 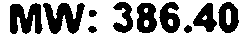 | | | | | |
| M22 | 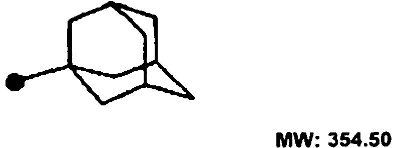 | 3.16 | 0.16 | 1.02 | 0.16 | 3.09 |
| M3* | 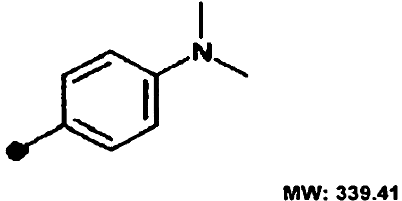 | 6.95 | 0.93 | 2.32 | 1.34 | 2.99 |
| M7* | 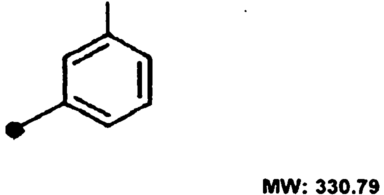 | 1.87 | 0.17 | 0.64 | 0.11 | 2.92 |
| M12* | 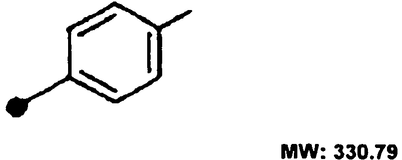 | 13.36 | 1.52 | 4.99 | 0.54 | 2.68 |
| M8 | 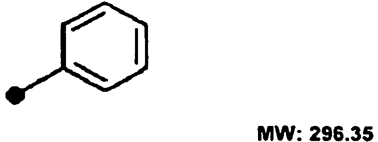 | 3.53 | 0.27 | 1.47 | 0.06 | 2.40 |
| M20* | 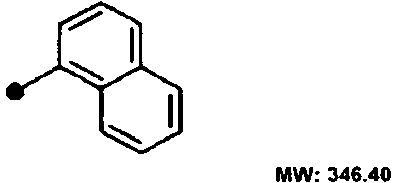 | 5.12 | 0.39 | 2.21 | 0.27 | 2.32 |
| M2 | 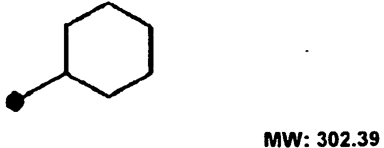 | 9.14 | 0.33 | 4.38 | 0.53 | 2.09 |
| M11 | 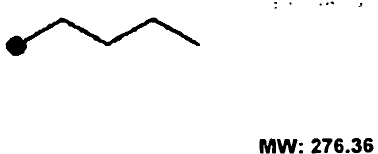 | 10.87 | 1.29 | 5.69 | 0.20 | 1.91 |
| M5* | 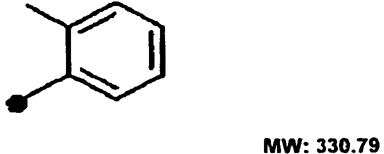 | 2.22 | 0.18 | 1.17 | 0.11 | 1.89 |
| M18* | 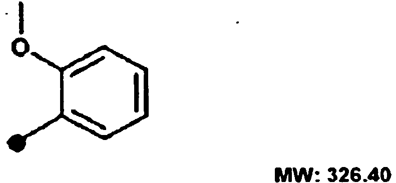 | 4.96 | 0.15 | 2.69 | 0.09 | 1.84 |
| M1 | 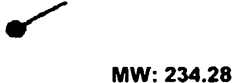 | 35.27 | 3.24 | 19.40 | 12.32 | 1.82 |
| M4 | 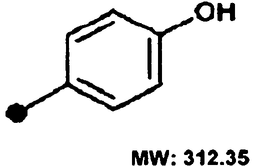 | 35.78 | 3.34 | 21.59 | 1.69 | 1.66 |
| M6 | 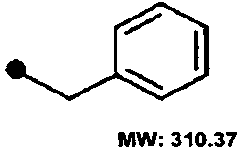 | 11.68 | 1.56 | 7.63 | 0.55 | 1.53 |
| M19* | 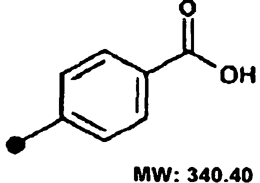 | >50 | - | >50 | - | 1.00 |

### EXAMPLES

The following examples are intended to be illustrative.

### General Methods

**Materials and methods**. Synthetic materials were sourced from Aldrich unless otherwise noted. Triapine (3-AP) was generously provided by Vion Pharmaceuticals, CT. MAIQ (NSC246112, 2-[(5-amino-4-methyl-1-isoquinolinyl)methylene]) was provided by the Developmental Therapeutics Program (DTP), National Institutes of Health. Thiacetazone was purchased from Sigma. Sunitinib was purchased from Toronto Chemicals, Toronto, Canada. Stock solutions of compounds for biological assays were prepared in DMSO and stored in frozen aliquots until use. The kinase inhibitory activity of NSC73306 against a panel of 50 kinases was measured by Reaction Biology Corp, Malvern, PA.

**Synthesis of thiosemicarbazones**. The thiosemicarbazones were prepared by combining equimolar quantities of the isatins and thiosemicarbazides dissolved in large amounts of ethanol with addition of a few drops of acetic acid to initiate the reaction. On heating the mixture to boiling the thiosemicarbazone often crystallized; if it did not, water was added to encourage it. The best solvent for recrystallization of thiosemicarbazones was DMSO with small amounts of water. As described above, the thiosemicarbazones also can be prepared by reacting t-Boc protected hydrazine, i-butylcarbazate, with a selected isothiocyanate resulting in i-Boc protected thiosemicarbazide that can be readily deprotected with acid.

With one exception (below), the (M+H)⁺ and (M-H)⁻ ions lost the elements of the corresponding RNCS on MS² although (M)⁻ ions were also detected. In the case of isatin allylthiosemicarbazone (Compound **13**), the (M+H)⁺ ion lost the elements of NHCSNHCH₂CHCH₃, likely as a five-membered thiourea-containing ring. The (M-H)⁻ ion lost allylisothiocyanate as expected. Low resolution mass spectra (LRMS) were collected on a Thermo LCQ Classic spectrometer (Madison, WI) and high resolution mass spectra (HRMS) with a Waters LCT Premier spectrometer (Milford, MA). ¹H NMR spectra were taken at 300 MHz in deuterated dimethylsulfoxide using a Varian 300 Gemini spectrometer (Palo Alto, CA).

### Example 1

### Synthesis and Characterization of MDR-Inverse compounds

This example describes the synthesis and characterization of several exemplary thiosemicarbazone compounds. Compounds **1** and **2** were prepared by condensation of 2-indanone and 1-indanone (in lieu of isatin) with 4-methoxyphenyl-3-thiosemicarbazide. Compound **3** was prepared by reacting thiosemicarbazide with isatin. The isatin-β-semicarbazones **4** and **14** were prepared by the reaction of the corresponding isatin with semicarbazide in lieu of their respective thiosemicarbazides. Compounds **7, 8, 9, 11** and **12** were prepared by condensation of the starting material (5-fluoroisatin, N-methyl isatin, sodium isatin-5-sulfonate, benz(g)indole-2,3-dione and 5-nitroisatin, respectively) with 4-methoxyphenyl-3-thiosemicarbazide.

**NSC73306. 1-Isatin-4-(4'metHoxyphenyl)thiosemicarbazone** was prepared by reacting isatin with 4-(4'methoxyphenyl)-3-thiosemicarbazide. Yield 87%, light yellow needles, mp 220-248°C dec., ¹H NMR (DMSO-*d6*) δ = 3.79 (3H, s), 6.98 (2H, dt), 6.98 (2H,dt), 7.45 (2H,dt), 6.95 (1H, d), 7.37 (1H,dt), 7.11 (1H, dt), 7.76 (1H, d), 12.7 (1H,s), 10.7 (1H,s), 11.25 (1H,s). LRMS *m*/*z* 327 (M+H)⁺, *m*/*z* 162 (MS2 of 327); *m*/*z* 326 (M)⁻, *m*/*z* 325 (M-H)⁻ *m*/*z* 160 (MS2 of 325) ; HRMS m/z 327.0917 (M+H)+, calc. C₁₆H₁₅N₄O₂S 327.0916.

**NSC716765**. **1-(5'-Nitroisatin)-4-allyl-3-thiosemicarbazone** was prepared by reacting 5-nitroisatin with 4-allyl-3-thiosemicarbazide. Yield 49%, light brown plates, mp 226-230°C dec. ¹H NMR (DMSO-*d6*) δ = 4.28 (2H, t), 5.18 (1H, d), 5.19 (1H, d), 5.92 (1H, m), 7.12 (1H, d), 8.27 (1H, dd), 8.57 (1H, d), 9.78 (1H, t), 11.83 (1H, bs), 12.38 (1H, s). LRMS m/z 306 (M+H)⁺, m/z 270 (MS2 of 306), m/z 304 (M-H)⁻, m/z 205 (MS2 of 304); HRMS m/z 306.0649 (M+H)+, calc. C₁₂H₁₂N₅O₃S 306.0661.

**NSC716766. 1-(5'-Nitroisatin)-4-phenyl-3-thiosemicarbazone** was prepared by reacting 5-nitroisatin with 4-phenyl-3-thiosemicarbazide. Yield 70%, light brown plates, mp 250-252°C dec. ¹H NMR (DMSO-*d6*) δ = 7.31 (1H, t), 7.45 (2H, t), 7.59 (2H, d), 7.17 (1H, d), 8.28 (1H, d), 8.70, 1H, d), 11.1 (1H, s), 12.55 (1H, s), 11.86 (1H, br s); LRMS m/z 342 (M+H)⁺, m/z 207 (MS2 of 342, m/z 340 (M-H)⁻, m/z 205 (MS2 of 340); HRMS m/z 342.0663 (M+H)+, calc. C₁₅H₁₂N₅O₃S 342.0661.

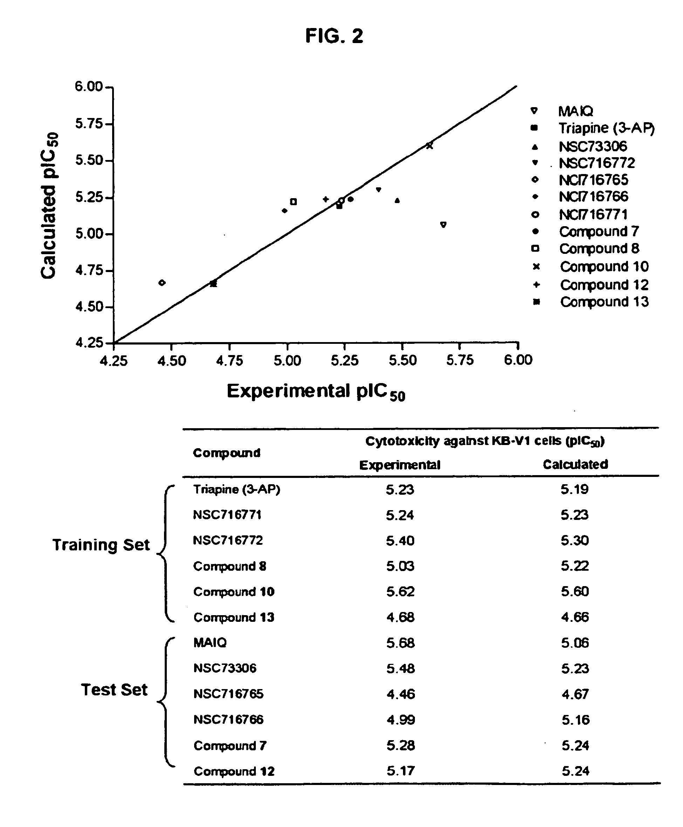

**1-(*t*-Butoxycarbonyl)-4-(4'-fluorophenyl)-3-thiosemicarbazide** (Figure 2c) was prepared by reaction of *t*-butylcarbazate with 4-fluorophenylisothiocyanate. Yield 90%, colorless prisms, m.p. 158-160°C. HRMS m/z 286.1019 (M+H)⁺, calc. C₁₂H₁₇N₃O₂FS 286.1026. The *t*-Boc group was removed by acid hydrolysis, and the resulting 4-(4'fluorophenyl)-3-thiosemicarbazide was used for the synthesis of NSC716771. Reaction of the simple hydrazine with 4-fluorophenylisothiocyanate yielded **1,2-Bis-(4'-fluorophenylthiocyanato)pydrazine** (Figure 2b). Yield 89%, colorless prisms, mp 204-205°C. HRMS m/z 339.0557 (M+H)⁺, calc. C₁₄H₁₃N₄F₂S₂ 339.0550; m/z 337.0388 (M-H)⁻, calc. C₁₄H₁₁N₄F₂S₂ 337.0393.

**NSC716771. 1-(5'-Bromoisatin)-4-(4'-fluorophenyl)-3-thiosemicarbazone** was prepared by reacting 5-bromoisatin with 4-(4'-fluorophenyl)-3-thiosemicarbazide. Yield 97%, orange needles, mp 238-240°C dec. ¹H NMR (DMSO-*d6*) δ = 6.91 (1 H, d), 7.53 (1H, dd), 7.97 (1H, d), 7.59 (2H, m), 7.28 (2H, t), 10.89 (1H, s), 11.36 (1H, s), 12.61 1H, s); HRMS m/z 392.9821 (M+H)+, calc. C₁₅H₁₁N₄OFSBr 392.9821.

**NSC716772. 1-(5'-Bromoisatin)-4-(4'-nitrophenyl)-3-thiosemicarbazone was** prepared by reacting 5-bromoisatin with 4-(4'-nitrophenyl)-3-thiosemicarbazide. Yield 83%, orange needles, mp 270-275°C dec., ¹H NMR (DMSO-*d*6) δ = 6.92 (1H, d), 7.55 (1H, dd), 8.00 (1H, d), 8.07 (2H, d), 8.31 (2H, d), 11.14 (1H, s), 11.41 (1H, s), 12.82 (1H, s); HRMS m/z 417.9608 (M+H)+, calc. C₁₅H₉N₅O₃SBr 417.9609.

**Compound 1. 1-(2'-Indanone)4-(4'-methoxyphenyl)-3-thiosemicarbazone** was prepared by reacting 2-indanone with 4-(4'-methoxyphenyl)-3-thiosemicarbazide. Yield 76%, white needles, browning in air, mp 152-4°C. ¹H NMR (DMSO-*d*6) δ = 3.75 (3H, s), 3.87 (4H, d), 6.82 (2H, d), 7.29 (2H, m), 7.32 (2H, m), 7.41 (2H, d), 9.81 (1H, s), 10.38 (1H, s); HRMS m/z 312.1179 (M+H)+, calc. C₁₇H₁₇N₃OS 312.1171.

**Compound 2. 1-(1'-Indanoue)-4-(4'-methoxyphenyl)-3-thiosemicarbazone** was prepared by reacting 1-indanone with 4-(4'-methoxyphenyl)-3-thiosemicarbazide. Yield 96%, white needles, mp 185-6°C. ¹H NMR (DMSO-*d*6) δ = 2.95 (2H, m), 3.08 (2H, m), 3.79 (3H, s), 6.92 (2H, d), 7.30 (1H, m), 7.38 (1H, d), 7.40 (1H, m), 7.41 (2H, d), 8.04 (1H, d), 9.99 (1H, s), 10.53 (1H, s); HRMS m/z 312.1179 (M+H)+, calc. C17H17N3OS 312.1171.

**Compound 3. 1-Isatin-3-thiosemicarbazone** was prepared by reacting isatin with 3-thiosemicarbazide. Yield 92%, fine yellow needles, mp 210-240°C dec. ¹H NMR (DMSO-*d*6) δ = 6.93 (1H,d), 7.09 (1H, t), 7.36 (1H, t), 7.65 (1H, d), 8.68 (1H, s), 9.03 (1H, s), 11.21 (1H, s), 12.47 (1H, s); LRMS m/z 221 (M+H)⁺, m/z 160 (MS2 of 221), m/z 219, (M-H)⁻, m/z 160 (MS2 of 219); HRMS m/z 219.0331 (M+H)⁺, calc. C₉H₇N₄SO 219.0340.

**Compound 4. 1-Isatin-3-semicarbazone** was prepared by reacting isatin with 3-semicarbazide. Yield 85%, light yellow needles, mp C dec., ¹H NMR (DMSO-*d*6) δ = 6.89 (1H, d), 6.91 (1H, s), 7.03 (1H, t), 7.34 (1H, t), 8.06 (1H, d), 10.18 (1H, s), 10.73 (1H, s); HRMS m/z 205.0707 (M+H)+, calc. C₉H₉N₄O₂ 205.0726. **Compound 5.4-(4'-Methoxyphenyl)-3-thiosemicarbazide** was obtained from Trans World Chemical (Rockville, MD). mp 154-156°C. ¹H NMR (DMSO-*d6*) δ 3.73 (3H, s), 4.76, (2H, br s), 6.86 (2H, d), 7.44 (2H, d), 8.72 (1H, br s), 9.50 (1H, br s).

**Compound 6. 1-(4',7'-Dichloroisatin)-4-(4'-methoxyphenyl)-3-thiosemicarbazone** was prepared by reacting 4,7-dichloroisatin with 4-(4'-methoxyphenyl)-3-thiosemicarbazide. Yield 89%, bright orange needles, mp 280°C dec., ¹H NMR (DMSO-*d*6) δ = 7.03 (2H, d), 7.55 (2H, d), 7.50 (1H, s), 7.19 (1H, s), 10.1 (2H, br s), 12.0 (1H, s); HRMS m/z 395.0142 (M+H)+, calc. C₁₆H₁₃N₄O₂SCl₂ 395.0136.

**Compound 7. 1-(5'-Fluoroisatin)-4-(4'-methoxyphenyl)-3-thiosemicarbazone** was prepared by reacting 5-fluoroisatin with 4-(4'-methoxyphenyl)-3-thiosemicarbazide. Yield 61%, orange needles, mp 238-240°C dec., ¹HNMR (DMSO-*d*6) δ = 3.79 (3H, s), 6.94 (1H, dd), 6.99 (2H, d), 7.21 (H, dt), 7.46 (2H, d), 7.62 (1H, dd), 10.78 (1H, s), 11.25 (1H, s), 12.63 (1H, s); LRMS m/z 345 (M+H)⁺, m/z 180 (MS2 of 345), m/z 343, (M-H)⁻, m/z 178, MS2 of 343; HRMS m/z 345.0830 (M+H)+, calc. C₁₆H₁₄N₄O₂FS 345.0822.

**Compound 8. 1-(*N*-methylisatin)-4-(4'-methoxyphenyl)-3-thiosemicarbazone** was prepared by reacting *N*-methylisatin with 4-(4'-methoxyphenyl)-3-thiosemicarbazide. Yield 87%, short yellow prisms, mp 216-17°C, ¹H NMR (DMSO-*d*6) δ = 3.78 (3H, s), 3.24, (3H, s), 6.98 (2H, d), 7.46 (2H, d), 7.16 (1H, d), 7.46 (1H, t), 7.17 (1H, t), 7.80 (1H, t), 10.75 (1h, s), 12.68 (1H, s); LRMS m/z 341 (M+H)⁺, m/z 176 (MS2 of 341), m/z 339 (M-H)⁻, m/z 174 (MS2 of 339). HRMS m/z 341.1078 (M+H)+, calc. C₁₇H₁₇N₄O₂S 341.1072.

**Compound 9. 1-(Isatin 5'-sulfonic acid)-4-(4'-methoxyphenyl)-3-thiosemicarbazone** was prepared by reacting isatin-5-sulfonic acid with 4-(4'-methoxyphenyl)-3-thiosemicarbazide. Yield 98%, dark yellow needles, mp > 300°C. ¹H NMR (DMSO-*d*6) δ = 3.79 (3H, s), 6.97 (2H, dd), 7.44 (2H, dd), 6.86

(1H, d), 7.62 (1H, dd), 8.13 (1H, br s), 10.95 (1H, s), 11.28 (1H, s), 12.70 (1H, s); LRMS no + or - ions. HRMS m/z 407.0469 (M+H)+, calc. C₁₆H₁₅N₄O₅S₂ 407.0469.

**Compound 10. 1-(2'-Pyridinecarboxaldehyde)-4-(4'-methoxyphenyl)-3-thiosemicarbazone** was prepared by reacting 2-pyridinecarboxaldehyde with 4-(4'-methoxyphenyl)-3-thiosemicarbazide. Yield 66%, mp 173-4°C, ¹NMR (DMSO-*d*6) δ = 3.76, (3H, s), 6.95 (2H, d), 7.39 (3H, d,m), 7.84 (1H, t), 8.18 (1H, s), 8.43 (1H,d), 8.58 (1H, d), 10.17 (1H, S), 11.95 (1H, s); HRMS m/z 287.0968 (M+H)+, calc. C₁₄H₁₅N₄OS 287.0967.

**Compound 11. 1-(1H'-Benz[g]indole-2',3'-dione)-4(4'-methoxyphenyl)-3-semicarbazone** was prepared by reacting 1H-Benz[g]indole-2,3-dione with 4-(4'-methoxyphenyl)-3-thiosemicarbazide. Yield 66%, dark red prisms, mp 240-268°C dec., ¹H NMR (DMSO-*d*6) δ = 3.79, (3H, s), 6.99 (2H, d), 7.48 (2H, m), 7.60 (2H, d), 7.66 (1 H, d). 7.88 (1H, d), 7.96 (1H, dd), 8.12 (1H, dd) 10.78 (1H, s), 11.97 (1H, s), 12.78 (1H, s); LRMS m/z 377 (M+H)⁺, m/z 212 (MS2 of 377), m/z 375 (M-H)⁻, m/z 210 (MS2 of 375); HRMS m/z 377.1073 (M+H)⁺, calc. C₂₀H₁₇N₄O₂S 377.1072.

**Compound 12. 1-(5'-Nitroisatin)-4-(4'-methoxyphenyl)-3-thiosemicarbazone** was prepared by reacting 5-nitroisatin with 4-(4'-methoxyphenyl)-3-thiosemicarbazide. Yield 63%, light yellow needles, mp 220-248°C dec., ¹H NMR (DMSO-*d*6) δ = 3.79 (3H, s), 7.00 (2H, d), 7.14 (1H, d), 7.44 (2H, d), 8.28 (1H, dd), 8.69 (1H, d), 11.01 (1H, s), 11.86 (1H, s), 12.51 (1H, s); LRMS m/z 372 (M+H)⁺, m/s 207 (MS2 of 372), m/s 370 (M-H)⁻, m/s 205 (MS2 of 370). HRMS m/z 372.0764 (M+H)+, calc. C₁₆H₁₄N_{S}O₄S 372.0767.

**Compound 13. 1-Isatin-4-allyl-3-thiosemicarbazone** was prepared by reacting isatin with 4-allyl-3-thiosemicarbazide. Yield 90%, light yellow needles, mp 210°C dec. ¹HNMR (DMSO-*d*6) δ = 4.25 (2H, t), 5.92 (1H, t), 5.18 (1H, m), 5.19 (1H, d), 6.93 (1H,dd), 7.35 (1H, dt), 7.09 (1H, dt), 7.67 (1H, dd), 9.45 (1H, t), 11.21 (1H, s), 12.60 (1H, s); LRMS no (M+H)⁺ ion, m/z 145 (loss of NHCSNHCH₂CH(CH₃)), m/z 259 (M-H)⁻, m/z 160 (MS2 of 259). HRMS m/z 261.0808 (M+H)+, calc. Cl₂H₁₃N₄OS 261.0810.

**Compound 14. 1-Isatin-4-(4'-methoxyphenyl)-3-semicarbazone** was prepared by reacting isatin with 4-(4'-methoxyphenyl)-3-semicarbazide. Yield 35%, yellow needles, mp 182-6°C, ¹NMR (DMSO-d6) δ = 3.74 (3H, s), 6.92 (2h, d), 7.49 (2H, d), 6.92 (1H, d), 7.39 (1H, m), 7.06 (1H, m), 8.09 (1H, d), 10.8 (1H, s); LRMS m/z 311 (M+H)⁺, m/z 162 (MS2 of 311), m/z 309 (M-H)⁻, m/z 160 (MS2 of 309); HRMS m/z 311.1138 (M+H)+, calc. C₁₆H₁₅N₄O₃ 311.1144.

**Compound 15. 1-(4'-Acetylaminobenzaldehyde)-4-(4'-methoxyphenyl)-3-thiosemicarbazone** was prepared by reacting 4-acetylaminobenzaldehyde with 4-(4'-methoxyphenyl)-3-thiosemicarbazide. Yield 98%, light yellow needles, mp 208-9°C, ¹NMR (DMSO-*d*6) δ = 2.05 (3H, s), 3.77 (3H, s), 6.92 (2H, d), 7.39 (2H, d), 7.63 (2H, d), 7.82 (2H, d), 9.93 (1H, s), 10.11 (1H, s), 11.68 (1H, s); HRMS m/z 343.1230 (M+H)⁺, calc. C₁₇H₁₉N₄O₂S 343.1229.

**Compound M13.** 2,4,6-trifluorophenylisothiocyanate was reacted with t-butylcarbazate. The t-Boc group was removed by acid hydrolysis, and the resulting product was 4-(2',4',6'-trifluorophenyl)-3-thiosemicarbazide. This product was reacted with isatin, yielding **1-Isatio-4-(2',4',6'-trifluorophenyl)thiosemicarbazone.**

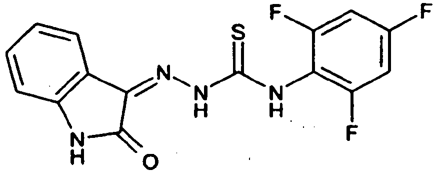

**Compound M14.** 4-fluorophenylisothiocyanate was reacted with t-butylcarbazate. The t-Boc group was removed by acid hydrolysis, and the resulting product was 4-(4'-fluorophenyl)-3-thiosemicarbazide. This product was reacted with isatin, yielding **1-Isatin-4-(4¹-fluorophenyl)thioscmicarbazone.**

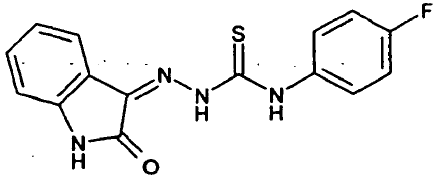

Compound M16. 4-methylphenylisothiocyanate was reacted with t-butylcarbazate. The t-Boc group was removed by acid hydrolysis, and the resulting product was 4-(4'-methylphenyl)-3-thiosemicarbazide. This product was reacted with isatin, yielding **1-Isatin-4-(4'-methylphenyl)thiosemicarbazone.**

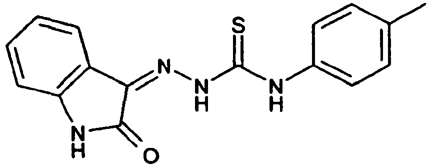

**Compound M17.** 4-nitrophenylisothiocyanate was reacted with t-butylcarbazate. The t-Boc group was removed by acid hydrolysis, and the resulting product was 4-(4'-nitrophenyl)-3-thiosemicarbazide. This product was reacted with isatin, yielding **1-Isatin-4-(4'-nitrophenyl)thiosemicarbazone.**

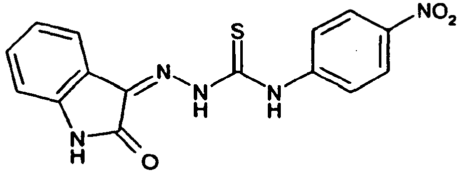

**Compound M1** was prepared by reaction of t-butylcarbazate with methyisothiocyanate. The t-Boc group was removed by acid hydrolysis, and the resulting 4-methyl-3-thiosemicarbazide was reacted with isatin, yielding **1-Isatin-4-methyl-3-thiosemicarbazone.**

**Compound M2** was prepared by reaction of t-butylcarbazate with cyclohexyl isothiocyanate. The t-Boc group was removed by acid hydrolysis, and the resulting 4-cyclohexyl-3-thiosemicarbazide was reacted with isatin, yielding **1-Isatin-4-cyclohexyl-3-thiosemicarbazone.**

**Compound M3** was prepared by reaction of t-butylcarbazate with 4-(dimethylamino)phenyl isothiocyanate. The t-Boc group was removed by acid hydrolysis, and the resulting 4'-(dimethylamino)phenyl-3-thiosemicarbazide was reacted with isatin, yielding **1-Isatin-4-(4'-(dimetbylamino)phenyl)-3-thiosemicarbazone.**

**Compound M4** was prepared by reaction of t-butylcarbazate with 4-hydroxyphenyl isothiocyanate. The t-Boc group was removed by acid hydrolysis, and the resulting 4-(4'-hydroxyphenyl)-3-thiosemicarbazide was reacted with isatin, yielding **1-Isatin-(4'-hydroxyphenyl)-3-thiosemicarbazone.**

**Compound M5** was prepared by reaction of t-butylcarbazate with 2-chlorophenyl isothiocyanate. The t-Boc group was removed by acid hydrolysis, and the resulting 4-(2'-chloroxyphenyl)-3-thiosemicarbazide was reacted with isatin, yielding 1-**Isatin-(2'-chlorophenyl)-3-thiosemicarbazone.**

**Compound M6** was prepared by reaction of t-butylcarbazate with benzyl isothiocyanate. The t-Boc group was removed by acid hydrolysis, and the resulting 4-benzyl-3-thiosemicarbazide was reacted with isatin, yielding **1-Isatin-4-benzyl-3-thiosemicarbazone.**

**Compound M7** was prepared by reaction of t-butylcarbazate with 3-chlorophenyl isothiocyanate. The t-Boc group was removed by acid hydrolysis, and the resulting 4-(3'-chloroxyphenyl)-3-thiosemicarbazide was reacted with isatin, yielding **1-Isatin-(3'-chlorophenyl)-3-thiosemicarbazone.**

**Compound M8** was prepared by reaction of t-butylcarbazate with phenyl isothiocyanate. The t-Boc group was removed by acid hydrolysis, and the resulting 4-(phenyl)-3-thiosemicarbazide was reacted with isatin, yielding **I-Isatingphenyl)-3-thiosemicarbazone.**

**Compound M9** was prepared by reaction of t-butylcarbazate with 4-(trifluoromethyl)phenyl isothiocyanate. The t-Boc group was removed by acid hydrolysis, and the resulting 4-(4'-(trifluoromethyl)phenyl)-3-thiosemicarbazide was reacted with isatin, yielding **1-Isatin-4-(4'-(tritluoromethyl)phenyl)-3-thiosemicarbazone.**

**Compound M10** was prepared by reaction of t-butylcarbazate with 4-fluorophenyl isothiocyanate. The t-Boc group was removed by acid hydrolysis, and the resulting 4-(4'- fluorophenyl)-3-thiosemicarbazide was reacted with 5-fluoroisatin, yielding **1-(5'-fluoroisatin)-4-(4'-fluorophenyl)-3-thiosemicarbazone.**

**Compound M11** was prepared by reaction of t-butylcarbazate with butyl isothiocyanate. The t-Boc group was removed by acid hydrolysis, and the resulting 4-butyl-3-thiosemicarbazide was reacted with isatin, yielding **1-Isatin-4-butyl-3-thiosemicarbazone.**

**Compound M12** was prepared by reaction of t-butylcarbazate with 4-chlorophenyl isothiocyanate. The t-Boc group was removed by acid hydrolysis, and the resulting 4-(4'-chloroxyphenyl)-3-thiosemicarbazide was reacted with isatin, yielding **1-Isatin-(4'-chloropbenyl)-3-thiosemicarbazone.**

**Compound M15** was prepared by reaction of t-butylcarbazate with isopropyl isothiocyanate. The t-Boc group was removed by acid hydrolysis, and the resulting 4-isopropyl-3-thiosemicarbazide was reacted with isatin, yielding **1-Isatin-4-isopropyl-3-thiosemicarbazone.**

**Compound M18** was prepared by reaction of t-butylcarbazate with 2-methoxyphenyl isothiocyanate. The t-Boc group was removed by acid hydrolysis, and the resulting 4-(2'-methoxyphenyl)-3-thiosemicarbazide was reacted with isatin, yielding **1-isatin-4-(2'-methoxypbenyl)-3-thiosemicarbazone.**

**Compound M19** was prepared by reaction of t-butylcarbazate with 4-carboxyphenyl isothiocyanate. The t-Boc group was removed by acid hydrolysis, and the resulting 4-(4'-carboxyphenyl)-3-thiosemicarbazide was reacted with 5-fluoroisatin, yielding **1-isatin-4-(4'-carboxyphenyl)-3-thiosemicarbazone.**

**Compound M20** was prepared by reaction of t-butylcarbazate with 1-napthyl isothiocyanate. The t-Boc group was removed by acid hydrolysis, and the resulting 4-(1'-napthyl)-3-thiosemicarbazide was reacted with isatin, yielding **1-isatin-4-(1'-napthyl)-3-thiosemicarbazone.**

**Compound M21** was prepared by reaction of t-butylcarbazate with 1-isothiocyanato-4-methoxybenzene. The t-Boc group was removed by acid hydrolysis, and the resulting 4-phenoxybenzene-3-thiosemicarbazide was reacted with isatin, yielding **1-Isatin-4-(4-phenoxybenzene)-3-thiosemicarbazone.**

**Compound M22** was prepared by reaction of t-butylcarbazate with 1-adamantyl isothiocyanate. The t-Boc group was removed by acid hydrolysis, and the resulting 4-(1'- adamantyl)-3-thiosemicarbazide was reacted with isatin, yielding **1-isatin-4-(1'- adamantyl)-3-thiosemicarbazone.**

**Compound M22** was prepared by reaction of t-butylcarbazate with 3,4,5-trimethoxyphenyl isothiocyanate. The t-Boc group was removed by acid hydrolysis, and the resulting 4-(3',4',5'-trimethoxyphenyl)-3-thiosemicarbazide was reacted with isatin, yielding **1-isatin-4-(3',4',5'-trimethoxyphenyl)-3-tbiosemicarbazone.**

### Example 2

### Assays for Identifying and Screening MDR-Inverse Compounds

This example describes evaluation of compounds using the MTT cytotoxicity assay. Cell survival was measured by the MTT (3-(4,5-dimethylthiazol-2-yl)-2,5-diphenyltetrazolium bromide) assays as previously described by Ludwig, J. A. et al. Selective toxicity of NSC73306 in MDR1-positive cells as a new strategy to circumvent multidrug resistance in cancer. Cancer Res 2006, 66, 4808-15, which is incorporated herein by reference. Briefly, cells were seeded in 100 mL of growth medium at a density of 5000 cells/well in 96-well plates and allowed to establish for 24 hours, at which time serially diluted drugs where added in an additional 100 mL growth medium. Cells were then incubated for 72 hours at 37°C in humidified 5% CO₂, at which time the growth media was drawn, and replaced with MTT in IMDM growth media and incubated for 4 hours. The MTT solution was then drawn from the wells, and 100 mL acidified ethanol solution was added to each well and after 15 minutes absorption at 560 nm was measured. IC₅₀ cytotoxicity values were determined as the drug concentration that reduced the absorbance to 50% of that in untreated control wells.

### Example 3

### Structure-Activity Relationships in MDR-Inverse Compounds

This example describes structure activity studies used to design exemplary compounds. Pharmacophore modeling and quantitative structure activity relationships (QSAR) were employed as two quantitative measures used to gauge the structural relationships of the compounds described above with respect to cytoxicity and MDR I-selectivity.

In this study, the generation of a pharmacophore for the cytotoxicity of compounds against the parental line KB-3-1 allowed for the possibility to determine what molecular components were required for cytotoxicity, compared with those required for MDR1-selectivity. The need for a thiosemicarbazone site is apparent, as compounds that lack this feature (**4** and **14**) proved to be relatively inactive. The thiosemicarbazone functional group was assigned a single site for two reasons; first, assigning individual bonding features of the thiosemicarbazone would potentially result in a large number of extra sites common to most molecules, and second it is possible the thiosemicarbazone group is coordinating to metal ions to effect its cytotoxicity and/or MDRI-selectivity as a bidentate ligand, as is the case for other classes of thiosemicarbazones (Liu et al. Chemical and biological properties of cytotoxic alpha-(N)-heterocyclic carboxaldehyde thiosemicarbazones. Prog Med Chem 1995, 31, 1-35).

The hydrogen bond acceptor site, corresponding to the aromatic ketone oxygen of isatin-β-thiosemicarbazones or the aromatic nitrogen of triapine, **10** and MAIQ, was deemed important for cytotoxicity, along with their associated aromatic ring/hydrophobic sites. Specific molecules assayed lacking any one of the thiosemicarbazone site, hydrogen bond acceptor site or aromatic ring/hydrophobic sites are inactive, and the failure of thiacetazones, **1, 2, 5** and **15** to match the KB-3-1 cytotoxicity pharmacophore can be understood in terms of missing the hydrogen bonding site. The use of a projected point for the hydrogen bond acceptor - simulating the corresponding hydrogen bond donor in the receptor- was introduced to allow structurally dissimilar active compounds to form hydrogen bonds to the same location, regardless of their point of origin and directionality. Such is the case with the isatin-β-thiosemicarbazones, triapine, MAIQ and **10**, in which the hydrogen bond acceptors originate in different locations of the ring/hydrophobic region, but are still capable of hydrogen bonding to a common site.

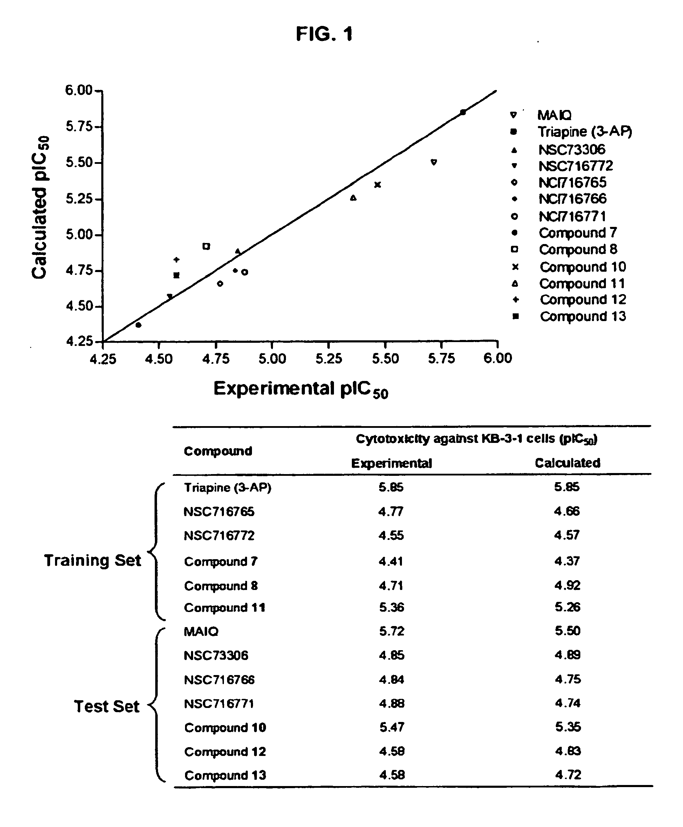

Apart from correctly identifying all of the active thiosemicarbazones, the KB-3-1 pharmacophore was used to pre-align the molecules for QSAR analysis. The performance of the single-factor atom-based QSAR model on the training and test set molecules is illustrated in FIG. 1. FIG. 1 is a scatter plot and comparison of the KB-3-1 cytoxicity QSAR model applied to thirteen active thiosemicarbazones. The training set correlation is characterized by one partial least-square (PLS) factor (SD = 0.12, R² = 0.96), which indicates exceptionally high correlation. The test set also exhibits exceptionally high correlation, being characterized by one PLS factor (Root mean-square error (RMSE) = 0.16, q² = 0.85, Pearson-R = 0.95). Experimental and calculated pIC₅₀ values are shown for the QSAR training and test set. The correlation coefficients are indicative of a model with strong predictive power and significance. FIG. 1 also compares experimental and predicted pIC₅₀ values for both the training and test set molecules, showing that activity was effectively predicted. This observation further supports the validity of the pharmacophore model, suggesting the spatial arrangement of chemical features, when aligned by the pharmacophore, is indicative of the probable active conformation of the molecule. The KB-3-1 cytoxicity QSAR model quantitatively predicts cytotoxicity, and although strong predictive power is evident, the low number of training/test set compounds warrant caution when using this model for this purpose. Of course, the predictive accuracy will improve once more compounds are added to training and test sets.

In defining the pharmacophore for the MDR1-inverse selectivity demonstrated by NSC73306, NSC716766, NSC716771, NSC716772, **7, 8, 10, 12,** and **13** (Table 1), three additional features were incorporated into the cytotoxicity pharmacophore described above; all represent electron-rich substitution at the N4 position of thiosemicarbazones common to MDR1-inverse compounds. These features, which match well with NSC73306, resulted in the MDR1-selectivity pharmacophore having seven sites. Setting a minimum of three sites for matching, corresponding to, the MDR1-selectivity pharmacophore can identify compounds that are cytotoxic but not necessarily selective. Incorporating further constraints by increasing the minimum number of required sites to the full seven descriptors, the pharmacophore highlights compounds that show selectivity for KB-V1 cells. Employing all seven sites predicts only compound **10** and the isatin-p-thiosemicarbazones possessing either a *p*-methoxyphenyl or *p*-fluorophenyl group at the N4 position. The performance of the single-factor atom-based QSAR model on the training and test set molecules for prediction of cytotoxicity against the MDR1 expressing KB-V1 line is illustrated in FIG. 2. FIG. 2 is a scatter plot and comparison of the KB-V1 cytoxicity QSAR model applied to twelve active thiosemicarbazones. The training set correlation is high, being characterized by one PLS factor (SD = 0.11, R² = 0.91). However, the test set correlation is lower, characterized by one PLS factor (RMSE = 0.29, q² = 0.42, Pearson-R = 0.70). Experimental and calculated pIC₅₀ values are shown for the QSAR training and test set. Experimental and predicted pIC₅₀ values against KB-V1 cells for both the training and test set molecules are also shown in FIG. 2, showing that activity was effectively predicted.

### Example 4

### Identification of Subjects having MDR Disorders

This example describes assays for identifying multidrug resistance in a subject. There are a variety of techniques to detect expression of MDR1. The detection and/or quantitation of MDR1 protein typically is accomplished using immunological techniques. For hematopoietic cells such as those from leukemia or lymphoma patients, the techniques include flow cytometry and fixed cells on microscope slides. The cells are treated with antibodies specific for the MDR1 protein, such as the mouse ARK-16 monoclonal antibody. Such antibodies can be directly labeled with fluorescent probe, or detected using subsequent reagents such as goat anti-mouse IgG-FITC. Flow cytometry allows for direct quantitative determinations of the full spectrum of MDR1 expression using channel number or fluorescence intensity. Microscopic examination of the slide preparations can give qualitative results (-, +, ++, and the like) or, in conjunction with an image analyzer, quantitative evaluations typically expressed in pixels.

For solid tumors, such as breast cancer, typically immunocytochemistry (ICC) or immunohistochemistry (IHC) techniques are employed. Using, for example, frozen sections or paraffin blocks, the detection techniques are the same as described for fixed leukemia cells on microscope slides. Expression of MDR1 can also be monitored by the measurement of specific mRNA levels. Cell slides can be processed, and levels of mRNA discerned using basic molecular biology techniques such as quantitative fluorescent PCR. Alternatively, the cells of interest can be lysed, processed, and following PCR of the mRNA, the product can be detected and quantitated following gel electrophoresis. Anti-sense targeting of MDR1 mRNA is also possible, followed by standard techniques for quantitative determinations. Radio-labeled probes followed by autoradiography or other radiodetection techniques can also be used to obtain a relative estimate of MDR1 protein or mRNA expression. Thus, there exists a broad range of methods for the detection and quantitation of the spectrum of MDR1 expression exhibited by a subject.

Monitoring of the relative expression of MDR1 is possible *in vivo.* MDR1-specific antibodies labeled with any number of detectable markers, such as radioactive compounds detectable with positron emission tomography (PET), single-photon emission computed tomography (ASPECT) or compounds detectable with magnetic resonance imaging (MRI) can be used to assess MDR1 expression in a subject having cancer or an MDR1-expressing infection, such as multidrug resistant tuberculosis.

MDR1 function, *i.e*., functional expression of MDR1 also can be evaluated. MDR1 functions as a cytoplasmic membrane pump, effluxing compounds such as drugs and toxins from the cytoplasm to the exterior of the cell. Compounds acted on by MDR1 are termed MDR1 substrates. Detection of MDR1 function therefore involves detection of substrate efflux, such as the efflux of a particular drug, or alternatively, detection of efflux of surrogate fluorescent dye markers that also are MDR1 substrates, such as DiOC2 (3,3'-diethyloxacarbocyanine iodide) or Rhodamine 123 (Rh123, or 2-(6-amino-3-imino-3H-xanthen-9-yl)benzoic acid, methyl ester). For single cell suspensions, such as blood or bone marrow from leukemia patients, the cells are exposed in tissue culture to a substrate for MDR1, such as the aforementioned dye markers, radiolabeled drugs, or drugs that can be detected and/or quantitated by other means such as fluorescence. At physiological temperature (37° C) the net accumulation of the substrate over time, in the presence or absence of specific MDR1 inhibitors, gives an indication of the MDR1 functional activity exhibited by the cells. Alternatively, the single cell suspension can be exposed to the substrate and subsequent efflux of the substrate over time monitored at physiological temperature in the presence or absence of specific MDR1 inhibitors.

PET, SPECT, and MRI techniques also can be used to assess MDR1 function in cancer patients. Thus, small organic molecules as well as metal complexes that serve as MDR1 substrates can be labeled with radionuclides or other detectable markers. Additionally, functional expression in solid tumors can be more efficiently ascertained by ICC/IHC techniques with prior labeling of the tumor cells in the patient.

Diagnostic testing methods for MDR1 expression and efflux pump activity can be used to prospectively stratify patients for treatment optimization in treating malignancies exhibiting MDR1 expression or function, such as acute myelogenous leukemia, most solid tumors, lymphomas, bladder cancer, pancreatic cancer, ovarian cancer, liver cancer, myeloma, lymphocytic leukemia, and sarcoma.

The disclosed techniques for identifying subjects having MDR-resistant cells are applicable to any therapeutic drug that is a substrate for P-gp-mediated efflux. Such drugs include, but are not limited to, P-glycoprotein substrates; anticancer drugs as described above and including, by way of example Vinca alkaloids such as vinblastine and vincristine; anthracyclines such as doxorubicin, daunorubicin, epirubicin; anthracenes such as bisantrene and mitoxantrone; epipodophyllo-toxins such as etoposide and teniposide; and other anticancer drugs such as actinomyocin D, mithomycin C, mitamycin, methotrexate, docetaxel, etoposide (VP-16), paclitaxel, docetaxel, and adriamycin; immunosuppressants, including cyclosporine A and tacrolimus; steroids, by way of example, dexamethasone, hydrocortisone, corticosterone, triamcinolone, aldosterone and methylprednisolone; antiepileptics, such as phenyloin; antidepressants, including without limitation, citalopram, thioperidone, trazodone, trimipramine, amitriptyline and phenothiazines; antipsychotics, such as fluphenazine, haloperidol, thioridazine and trimipramine; HIV protease inhibitors, for example, amprenavir, indinavir, lopinavir, nelfinavir, ritonavir and saquinavir; calcium blockers, for example, bepridil, diltiazem, flunarizine, lomerizine, secoverine, tamolarizine, verapamil, nicardipine, prenylamine and fendiline.

### Example 5

### Monotherapy using MDR-Inverse Compounds

This example describes the treatment of a subject having a multidrug resistant disorder, such as a multidrug resistant tumor. Subjects having such disorders can be identified, for example, as set forth above in Example 4. In one embodiment, a subject having a multidrug resistant disorder is administered an MDR-inverse compound disclosed herein, such as compound 7:

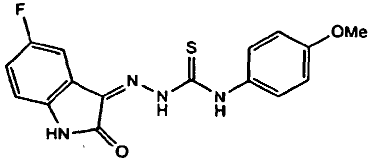

### Compound 7

in an amount sufficient to elevate the target tissue concentration of the MDR-inverse compound, such as compound 7, in the subject to at least about 10 nM, such as from about 0.1 µM to about 100 µM, and typically from about 1 µM to about 10 µM. One skilled in the art will recognize that other MDR-inverse compounds disclosed herein can be administered in place of or in addition to compound 7. In one embodiment, the MDR-inverse compound is administered intravenously in an amount of 400 mg/day or less to about 1,600 mg/day or more, preferably from about 500, 600, or 700 mg/day to about 900, 1000, 1100, 1200, 1300, 1400, or 1500 mg/day, and most preferably about 700 mg/day. In the course of a treatment regimen, the MDR-inverse compound, such as compound 7, preferably is administered on two, three, or four separate days. The dosage typically is administered in intravenously continuously over the course of about 3 to about 90 hours, more preferably over the course of about 4, 6, 12, 18, 24 or 30, 36, or 42 hours to about 54, 60, 66, 72, 78, or 84 hours, most preferably over about 24 hours, 48 hours, or 72 hours, depending upon the treatment regimen. Preferably the MDR-inverse compound is administered on multiple days of the treatment regimen.

### Example 6

### Combination Therapy using MDR-Inverse Compounds

As discussed above, the drugs which are substrates of P-gp are quite varied as are the associated disease states. One form of cancer characterized by high rates of P-gp expression is acute myelogenous leukemia. This example describes the treatment of acute myelogenous leukemia, where it has been demonstrated that the levels of P-gp expression and function are significantly related to response to the chemotherapy.

Standard induction therapy in the U.S. for newly diagnosed acute myelogenous leukemia patients is cytarabine with either idarubicin or daunorubicin (both P-gp substrates). Daunorubicin is an antibiotic chemotherapy treatment that is widely used to treat acute myeloid leukemia and acute lymphocytic leukemia. It was approved by the FDA as a first line therapy treatment for leukemia in 1998. Daunorubicin is typically administered intravenously. Daunorubicin is marketed under the brand names Cerubidine, DaunoXome, and Liposomal daunorubicin.

Cytarabine is a deoxycytidine analogue, cytosine arabinoside (ara-C), which is metabolically activated to the triphosphate nucleotide (ara-CTP), which acts as a competitive inhibitor of DNA polymerase and produces S phase-specific cytotoxicity. It is used as an antineoplastic, generally as part of a combination chemotherapy regimen, in the treatment of acute lymphocytic and acute myelogenous leukemia, the blast phase of chronic myelogenous leukemia, erythroleukemia, and non-Hodgkin's lymphoma. The compound typically is administered intravenously and subcutaneously, and for the prophylaxis and treatment of meningeal leukemia, administered intrathecally.

Contemplated amounts of the presently disclosed MDR-inverse compounds, such as NSC73306, for administration to treat acute myelogenous leukemia are from about 400 mg/day or less to about 1,600 mg/day or more, preferably from about 500, 600, or 700 mg/day to about 900, 1000, 1100, 1200, 1300, 1400, or 1500 mg/day, and most preferably about 700 mg/day. In the course of a treatment regimen, the MDR-inverse compound, such as NSC73306, preferably is administered on two, three, or four separate days. One skilled in the art will recognize that other MDR-inverse compounds disclosed herein can be administered in place of or in addition to NSC73306. The dosage typically is administered in intravenously continuously over the course of about 6 to about 90 hours, more preferably over the course of about 12, 18, 24 or 30, 36, or 42 hours to about 54, 60, 66, 72, 78, or 84 hours, most preferably over about 24 hours, 48 hours, or 72 hours, depending upon the treatment regimen. Preferably the MDR-inverse compound is administered on multiple days of the treatment regimen.

Contemplated amounts of daunorubicin for intravenous administration to treat acute myelogenous leukemia are from about 10 mg/m²/day or less to about 100 mg/m²/day or more administered in combination with MDR-inverse compound infusion or up to about 1 to about 8, such as 1, 2, 3, 4, 5, or 6 or more hours after initiation of MDR-inverse compound infusion. The dosage is preferably administered intravenously at a rate of about 25 mg/m²/day or less to about 90 mg/m²/day or more, preferably about 30, 35, or 40 mg/m²/day or less to about 50, 55, 60, 65, 70, 75, 80, or 85 mg/m²/day, and most preferably about 45 mg/m²/day continuously over the course of about 2 or 2.5 days to about 3.5 or 4 days, preferably about 3 days.

Amounts of cytarabine for intravenous administration to treat acute myelogenous leukemia are from about 10 mg/day or less to about 3,000 mg/day or more administered at initiation of MDR-inverse compound infusion or after initiation of MDR-inverse compound infusion. The dosage is preferably administered intravenously at a rate of about 50 mg/m²/day or less to about 200 mg/m²/day or more, preferably 60, 70, 80, or 90 mg/m²/day or less to about 110, 120, 130, 140, 150, 160, 170, 180, or 190 mg/m.sup.2/day, and most preferably about 100 mg/m²/day continuously over the course of about 1, 2, 3, 4, 5, or 6 days up to about 8, 9, or 10 days or more, preferably over about 7 days.

While the above methods of the preferred embodiments have been discussed primarily in connection with the treatment of acute myelogenous leukemia, the methods are also particularly effective when P-gp substrates are administered as chemotherapeutic agents in the treatment of other disorders, including other hyperproliferative disorders, exhibiting some degree of P-gp expression. For example, such disorders can include lymphomas, bladder cancer, pancreatic cancer, ovarian cancer, liver cancer, myeloma, lymphocytic leukemia, sarcoma, metastatic breast cancer, and most solid tumors. Chemotherapeutic agents that are P-gp substrates include, without limitation, anthracyclines (for example, doxorubicin, daunorubicin, epirubicin, idarubicin, mitoxantrone), Vinca alkaloids (for example, vincristine, vinblastine, vinorelbine, vindesine), Topoisomerase-II inhibitors (for example, etoposide, teniposide), taxanes (e.g., paclitaxel, docetaxel), and others (for example, Gleevec and dactinomycin).

## Claims

1. A compound according to the formula 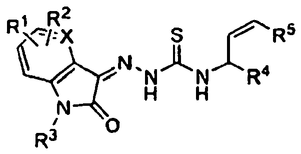 wherein R¹ and R² independently are selected from H, fluoro,and iodo;
X is CH;
R³ is H;
R⁴ is H; or together with R⁵ forms an optionally substituted phenyl or naphthyl ring;
R⁵ is H; or together with R⁴ forms an optionally substituted phenyl or naphthyl ring;
and
when R⁴ and R⁵ form a *para*-methoxyphenyl moiety, at least one of R¹, or R² is other than H;
provided that the compound is not one of the following compounds: 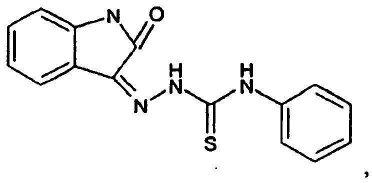 or 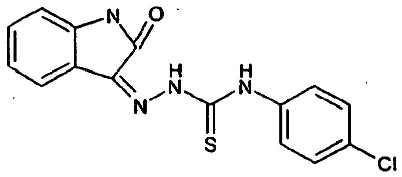 for use in a method of inhibiting the growth of drug resistant cells in a subject, comprising identifying a subject having drug resistant cells and administering the compound to the subject.

2. The compound for use as in claim 1, wherein the compound has the formula 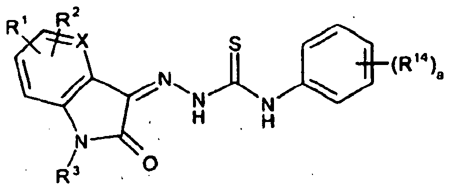 wherein a is 0 to 5;
R¹⁴ is halogen; -OR¹⁵; -NR¹⁶R¹⁷; cyano; nitro; haloalkyl; lower alkyl; or carboxy, and each R¹⁴ may be the same or different;
R¹⁵ is lower alkyl;
R¹⁶ is lower alkyl; and
R¹⁷ is lower alkyl;
wherein the lower alkyl is methyl, ethyl, n-propyl, isopropyl, n-butyl, isobutyl, *t-*butyl, pentyl, hexyl, heptyl, octyl or decyl.

3. The compound for use as in claim 1, wherein the compound has the formula 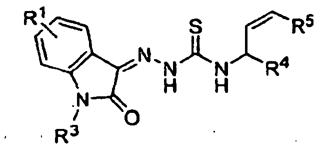 wherein R¹, R³, R⁴ and R⁵ are as set forth above; or wherein the compound has the formula

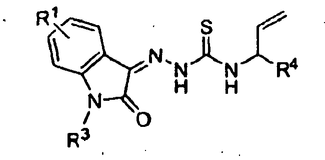

4. The compound for use as in claim 1, wherein the compound has the formula 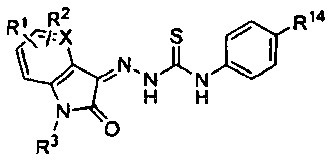 wherein R¹⁴ is H; halogen; -OR¹⁵; -NR¹⁶R¹⁷; cyano; nitro; haloalkyl; lower alkyl; or carboxy; or wherein the compound has the formula 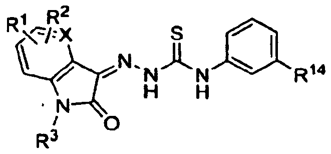 wherein R¹⁴ is H; halogen; -OR¹⁵; -NR¹⁶R¹⁷; cyano; nitro; haloalkyl; lower alkyl; or carboxy; or wherein the compound has the formula 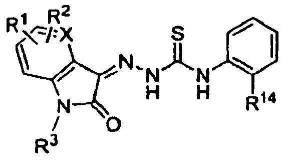 wherein R¹⁴ is H; halogen; -OR¹⁵; -NR¹⁶R¹⁷; cyano; nitro; haloalkyl; lower alkyl; or carboxy;
wherein the lower alkyl is methyl, ethyl, *n*-propyl, isopropyl, *n*-butyl, isobutyl, *t-*butyl, pentyl, hexyl, heptyl, octyl or decyl

5. The compound for use as in claims 2 to 4, wherein R¹⁴ is -OMe; or wherein R¹⁴ is haloalkyl.

6. The compound for use as in claim 2, wherein R¹⁴ is fluoro or fluoroalkyl.

7. The compound for use as in claim 1, wherein the compound has the formula 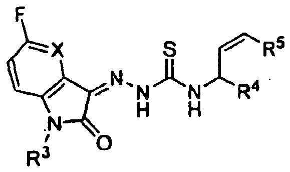 or wherein the compound has the formula 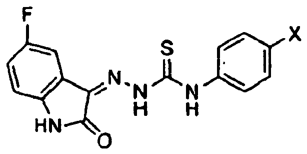 wherein X is selected from H, -OCH₃, -CH₃, -CH₂CH₃, -F, -Cl, -Br, -I, - CF₃, -OCF₃, -NO₂, phenyl, -N₃, -CN, -OH, -NH₂, -NMe₂, -COOH and -SO₃ ; or wherein the compound has the formula 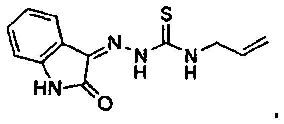 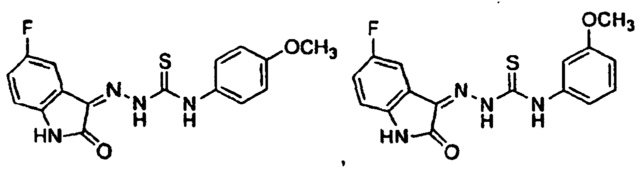 or 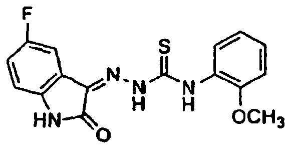 or wherein the compound has the formula 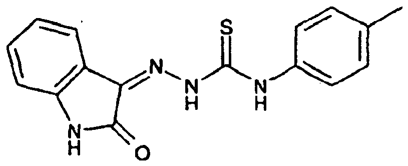 or wherein the compound has the formula 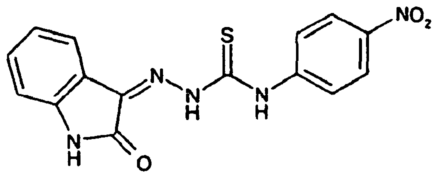 or wherein the compound has the formula 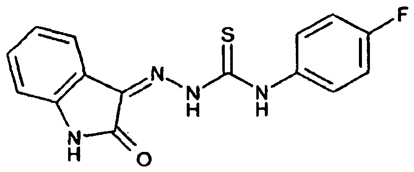 or wherein the compound has the formula

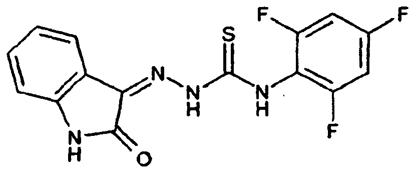

8. The compound for use as in any of claims 1 to 7, wherein the cells exhibit a Multidrug resistance phenotype; or further comprising re-sensitizing the cells to an MDR1 substrate; or wherein the cells are neoplastic cells.

9. The compound for use as in any of claims 1 to 8, wherein the cells comprise cancer; particularly, wherein the cancer comprises brain cancer, breast cancer, bladder cancer, bone cancer, cervical cancer, colon cancer, central nervous system cancer, esophageal cancer, gall bladder cancer, gastrointestinal cancer, head and neck cancer, Hodgkin's Disease, non-Hodgkin's lymphomas, laryngeal cancer, leukemia, lung cancer, melanoma, neuroblastoma, ovarian cancer, pancreatic cancer, prostate cancer, rectal cancer, renal cancer, retinoblastoma, stomach cancer, testicular cancer, Wilms' tumor or a combination thereof.

10. The compound for use as in claim 1, wherein the method comprises inhibiting the development of multidrug resistance in the hyperproliferative disorder; or wherein the subject has previously been treated with at least one MDR1 substrate.

11. The compound for use as in any of claims 1 to 10, wherein the compound is administered in combination with a cytotoxic agent; particularly, wherein the cytotoxic agent is an anticancer agent; more particularly, wherein the anticancer agent is selected from the microtubule binding agents, DNA intercalators, DNA alkylating agents, DNA cross-linkers, DNA synthesis inhibitors, DNA and/or RNA transcription inhibitors, enzyme inhibitors, gene regulators, enzymes, antibodies and angiogenesis inhibitors; yet more particularly, wherein the anticancer agent is selected from erlotinib, gefitinib, temozolomide, paclitaxel, docetaxel, daunorubicin, cisplatin, carboplatin, oxaliplatin, colchicine, dolastatin 15, nocodazole podophyllotoxin, rhizoxin, vinblastine, vindesine, vinorelbine (navelbine), the epothilones, the mitomycins, bleomycin, chlorambucil, carmustine, melphalan, mitoxantrone, 5-fluoro-5'-deoxyuridine, camptothecin, SFTI-1, topotecan, irinotecanetoposide, tenoposide, geldanamycin, methotrexate, adriamycin, actinomycin D, medroxyprogesterone, mifepristone, raloxifene, 5-azacytidine, 5-aza-2'-deoxycytidine, zebularine, tamoxifen, 4-hydroxytamoxifen, apigenin, rapamycin, angiostatin K1-3, L-asparaginase, staurosporine, genistein, fumagillin, endostatin, isophosphoramide mustard, thalidomide and analogs thereof.

12. A composition comprising an amount of the compound of any of claims 1 to 11 effective to inhibit the growth of neoplastic cells in a subject and a pharmaceutically acceptable carrier for use in a method of inhibiting the growth of drug resistant cells in the subject, comprising identifying a subject having drug resistant cells and administering the compound to the subject; particularly wherein the composition further comprises an effective amount of an anticancer agent selected from the microtubule binding agents, DNA intercalators, DNA alkylating agents, DNA cross-linkers, DNA synthesis inhibitors, DNA and/or RNA transcription inhibitors, enzyme inhibitors, gene regulators, enzymes, antibodies and angiogenesis inhibitors.

13. A compound according to the formula 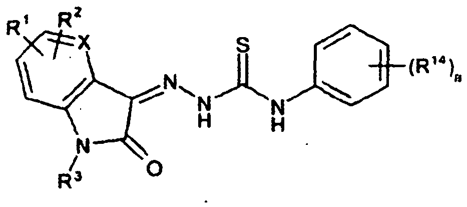 wherein a is 0 to 5;
wherein R¹ and R¹ independently are selected from H; and iodo;
X is CH;
R³ is H;
and
R¹⁴ is fluoro; iodo; -OR¹⁵; NR¹⁶R¹⁷; cyano; haloalkyl; carboxy; ethyl, n-propyl, isopropyl, *n*-butyl, isobutyl, *t*-butyl, pentyl, hexyl, heptyl, octyl or decyl; and each R¹⁴ may be the same or different;
R¹⁵ is ethyl, n-propyl, isopropyl, n-butyl, isobutyl, t-butyl, pentyl, hexyl, heptyl, octyl or decyl;
R¹⁶ is lower alkyl; and
R¹⁷ is lower alkyl,;
wherein the lower alkyl is methyl, ethyl, n-propyl, isopropyl, n-butyl, isobutyl, t-butyl, pentyl, hexyl, heptyl, octyl or decyl; or a compound according to the formula: 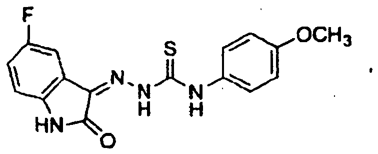 provided that the compound is not:

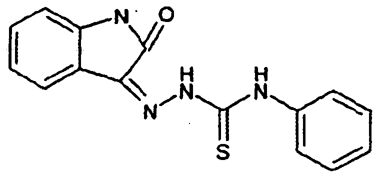

14. The compound of claim 13 wherein R¹⁴ is fluoro or haloalkyl, particularly fluoroalkyl; particularly, wherein R¹⁴ is fluoro or fluoroalkyl; X is CH: and R₁, R₂ and R₃ are each H; more particularly, wherein the compound has the formula 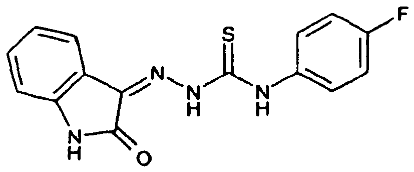 or wherein the compound has the formula

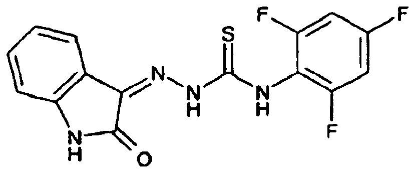

15. A composition comprising an amount of the compound of any of claims 12 to 14 effective to inhibit the growth of neoplastic cells in a subject and a pharmaceutically acceptable carrier; particularly, wherein the composition further comprises an effective amount of an anticancer agent selected from the microtubule binding agents, DNA intercalators, DNA alkylating agents, DNA cross-linkers, DNA synthesis inhibitors, DNA and/or RNA transcription inhibitors, enzyme inhibitors, gene regulators, enzymes, antibodies and angiogenesis inhibitors.

16. The compound for use as in claims 2, 5 to 6, 8 or 9, wherein subscript a is 1.

17. The compound for use as in claims 2, 5 to 6, 8 or 9, wherein subscript a is 2 to 5.

18. The compound of claim 13 or 14, wherein subscript a is 1.

19. The compound of claim 13 or 14, wherein subscript a is 2 to 5.

## Patentansprüche

1. Verbindung gemäß der Formel 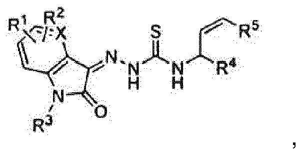 wobei R¹ und R² unabhängig aus H, Fluor und lod ausgewählt sind;
X CH ist;
R³ H ist;
R⁴ H ist oder zusammen mit R⁵ einen gegebenenfalls substituierten Phenyl- oder Naphthylring bildet;
R⁵ H ist oder zusammen mit R⁴ einen gegebenenfalls substituierten Phenyl- oder Naphthylring bildet;
und
wenn R⁴ und R⁵ eine para-Methoxyphenyleinheit bilden, mindestens eines von R¹ oder R² etwas anderes als H ist;
vorausgesetzt, dass die Verbindung nicht eine der folgenden Verbindungen ist: 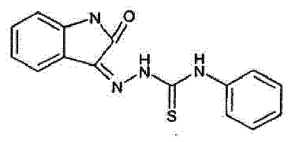 oder 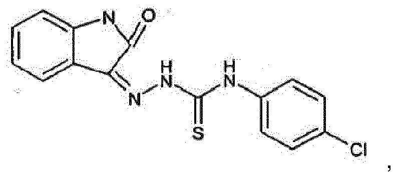 zur Verwendung in einem Verfahren zum Hemmen des Wachstums von arzneimittelresistenten Zellen in einer Person, wobei das Verfahren das Identifizieren einer Person mit arzneimittelresistenten Zellen und das Verabreichen der Verbindung an die Person umfasst.

2. Verbindung zur Verwendung nach Anspruch 1, wobei die Verbindung die folgende Formel aufweist: 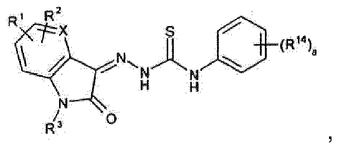 wobei a 0 bis 5 ist;
R¹⁴ Halogen; -OR¹⁵; -NR¹⁶R¹⁷; Cyano; Nitro; Halogenalkyl; Niederalkyl oder Carboxy ist und jedes R¹⁴ gleich oder verschieden sein kann;
R¹⁵ Niederalkyl ist;
R¹⁶ Niederalkyl ist und
R¹⁷ Niederalkyl ist;
wobei das Niederalkyl Methyl, Ethyl, *n*-Propyl, Isopropyl, *n*-Butyl, Isobutyl, *t-*Butyl, Pentyl, Hexyl, Heptyl, Octyl oder Decyl ist.

3. Verbindung zur Verwendung nach Anspruch 1, wobei die Verbindung die folgende Formel aufweist: 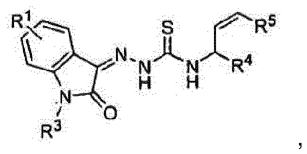 wobei R¹, R³, R⁴ und R⁵ wie oben dargelegt sind; oder wobei die Verbindung die folgende Formel aufweist:

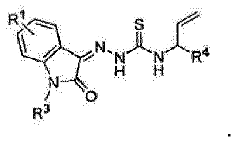

4. Verbindung zur Verwendung nach Anspruch 1, wobei die Verbindung die folgende Formel aufweist: 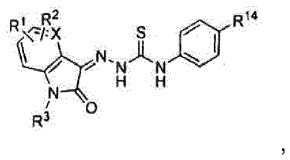 wobei R¹⁴ H; Halogen; -OR¹⁵; -NR¹⁶R¹⁷; Cyano; Nitro; Halogenalkyl; Niederalky oder Carboxy ist; oder wobei die Verbindung die folgende Formel aufweist: 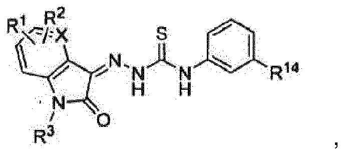 wobei R¹⁴ H; Halogen; -OR¹⁵; -NR¹⁶R¹⁷; Cyano; Nitro; Halogenalkyl; Niederalkyl oder Carboxy ist; oder wobei die Verbindung die folgende Formel aufweist: 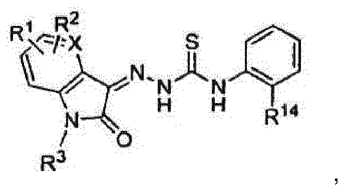 wobei R¹⁴ H; Halogen; -OR¹⁵; -NR¹⁶R¹⁷; Cyano; Nitro; Halogenalkyl; Niederalkyl oder Carboxy ist;
wobei das Niederalkyl Methyl, Ethyl, *n*-Propyl, Isopropyl, *n*-Butyl, Isobutyl, *t-*Butyl, Pentyl, Hexyl, Heptyl, Octyl oder Decyl ist.

5. Verbindung zur Verwendung nach den Ansprüchen 2 bis 4, wobei R¹⁴ -OMe ist oder wobei R¹⁴ Halogenalkyl ist.

6. Verbindung zur Verwendung nach Anspruch 2, wobei R¹⁴ Fluor oder Fluoralkyl ist.

7. Verbindung zur Verwendung nach Anspruch 1, wobei die Verbindung die folgende Formel aufweist: 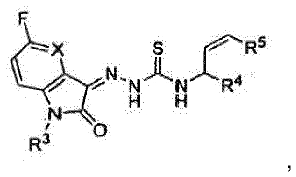 oder wobei die Verbindung die folgende Formel aufweist: 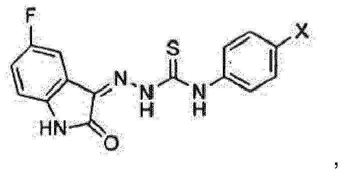 wobei X aus H, -OCH₃, -CH₃, -CH₂CH₃, -F, -Cl, -Br, -I, -CF₃, -OCF₃, -NO₂, Phenyl, -N₃, -CN, -OH, -NH₂, -NMe₂, -COOH und -SO₃ ausgewählt ist; oder
wobei die Verbindung die folgende Formel aufweist: 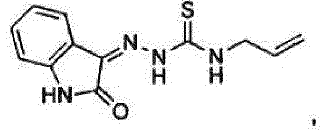 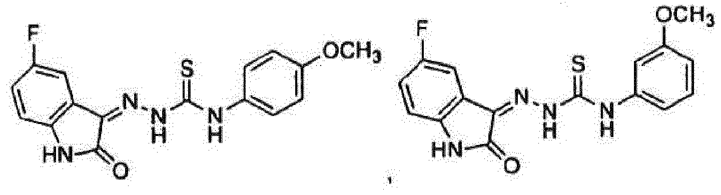 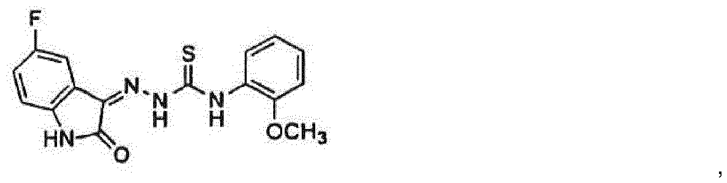 oder wobei die Verbindung die folgende Formel aufweist: 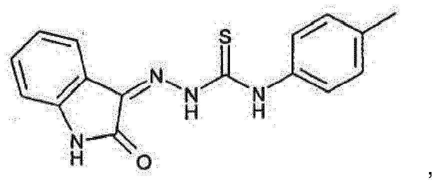 oder wobei die Verbindung die folgende Formel aufweist: 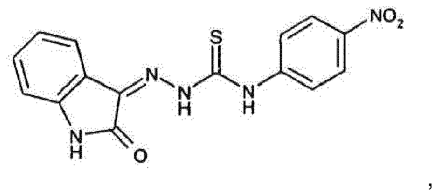 oder wobei die Verbindung die folgende Formel aufweist: 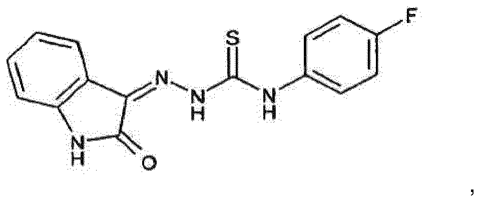 oder wobei die Verbindung die folgende Formel aufweist:

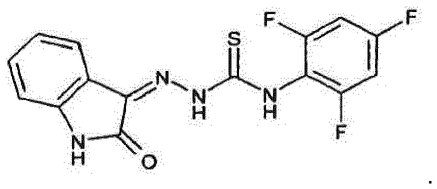

8. Verbindung zur Verwendung nach einem der Ansprüche 1 bis 7, wobei die Zellen einen Multidrug-Resistenz-Phänotyp aufweisen; oder die weiterhin das Resensibilisieren der Zellen für ein MDR1-Substrat umfasst; oder wobei die Zellen neoplastische Zellen sind.

9. Verbindung zur Verwendung nach einem der Ansprüche 1 bis 8, wobei die Zellen Krebs umfassen; insbesondere wobei der Krebs Hirnkrebs, Brustkrebs, Blasenkrebs, Knochenkrebs, Gebärmutterhalskrebs, Dickdarmkrebs, Krebs des Zentralnervensystems, Speiseröhrenkrebs, Gallenblasenkrebs, Magen-Darm-Krebs, Kopf- und Halskrebs, Morbus Hodgkin, non-Hodgkin-Lymphome, Kehlkopfkrebs, Leukämie, Lungenkrebs, Melanom, Neuroblastom, Eierstockkrebs, Bauchspeicheldrüsenkrebs, Prostatakrebs, Rektumkrebs, Nierenkrebs, Retinoblastom, Magenkrebs, Hodenkrebs, Wilms-Tumor oder eine Kombination davon umfasst.

10. Verbindung zur Verwendung nach Anspruch 1, wobei das Verfahren das Hemmen der Entwicklung von Multidrug-Resistenz in der hyperproliferativen Erkrankung umfasst oder wobei die Person zuvor mit mindestens einem MDR1-Substrat behandelt wurde.

11. Verbindung zur Verwendung nach einem der Ansprüche 1 bis 10, wobei die Verbindung in Kombination mit einem Zytotoxikum verabreicht wird; insbesondere wobei das Zytotoxikum eine antineoplastische Substanz ist; ganz besonders wobei die antineoplastische Substanz aus den Mikrotubuli bindenden Mitteln, DNA-Interkalatoren, DNA-Alkylierungsmitteln, DNA-Vernetzern, DNA-Synthesehemmern, DNA- und/oder RNA-Transkriptionshemmern, Enzymhemmern, Genregulatoren, Enzymen, Antikörpern und Angiogenesehemmern ausgewählt ist; noch mehr besonders wobei die antineoplastische Substanz aus Erlotinib, Gefitinib, Temozolomid, Paclitaxel, Docetaxel, Daunorubicin, Cisplatin, Carboplatin, Oxaliplatin, Colchicin, Dolastatin 15, Nocodazol-Podophyllotoxin, Rhizoxin, Vinblastin, Vindesin, Vinorelbin (Navelbin), den Epothilonen, den Mitomycinen, Bleomycin, Chlorambucil, Carmustin, Melphalan, Mitoxantron, 5-Fluor-5'-desoxyuridin, Camptothecin, SFTI-1, Topotecan, Irinotecanetoposid, Tenoposid, Geldanamycin, Methotrexat, Adriamycin, Actinomycin D, Medroxyprogesteron, Mifepriston, Raloxifen, 5-Azacytidin, 5-Aza-2'-desoxycytidin, Zebularin, Tamoxifen, 4-Hydroxytamoxifen, Apigenin, Rapamycin, Angiostatin K1-3, L-Asparaginase, Staurosporin, Genistein, Fumagillin, Endostatin, Isophosphoramid-Mustard, Thalidomid und Analoga davon ausgewählt ist.

12. Zusammensetzung, die eine Menge der Verbindung nach einem der Ansprüche 1 bis 11, die zum Hemmen des Wachstums von neoplastischen Zellen in einer Person wirksam ist, und einen pharmazeutisch unbedenklichen Trägerstoff umfasst, zur Verwendung in einem Verfahren zum Hemmen des Wachstums von arzneimittelresistenten Zellen in der Person, wobei das Verfahren das Identifizieren einer Person mit arzneimittelresistenten Zellen und das Verabreichen der Verbindung an die Person umfasst; insbesondere wobei die Zusammensetzung weiterhin eine wirksame Menge einer antineoplastischen Substanz umfasst, die aus den Mikrotubuli bindenden Mitteln, DNA-Interkalatoren, DNA-Alkylierungsmitteln, DNA-Vernetzern, DNA-Synthesehemmern, DNA- und/oder RNA-Transkriptionshemmern, Enzymhemmern, Genregulatoren, Enzymen, Antikörpern und Angiogenesehemmern ausgewählt ist.

13. Verbindung gemäß der Formel 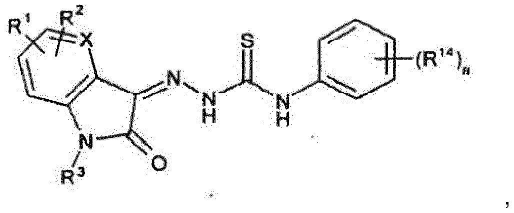 wobei a 0 bis 5 ist;
wobei R¹ und R² unabhängig aus H und lod ausgewählt sind;
X CH ist;
R³ H ist;
und
R¹⁴ Fluor; lod; -OR¹⁵; -NR¹⁶R¹⁷ ; Cyano; Halogenalkyl; Carboxy; Ethyl, *n*-Propyl, Isopropyl, *n*-Butyl, Isobutyl, *n*-Butyl, Pentyl, Hexyl, Heptyl, Octyl oder Decyl ist und jedes R¹⁴ gleich oder verschieden sein kann;
R¹⁵ Ethyl, *n*-Propyl, Isopropyl, *n*-Butyl, Isobutyl, *t*-Butyl, Pentyl, Hexyl, Heptyl, Octyl oder Decyl ist;
R¹⁶ Niederalkyl ist und
R¹⁷ Niederalkyl ist;
wobei das Niederalkyl Methyl, Ethyl, *n*-Propyl, Isopropyl, *n*-Butyl, Isobutyl, *t-*Butyl, Pentyl, Hexyl, Heptyl, Octyl oder Decyl ist; oder Verbindung gemäß der Formel: 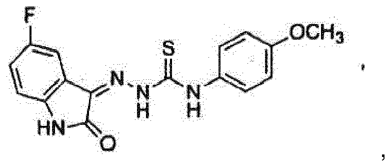 vorausgesetzt, dass die Verbindung nicht 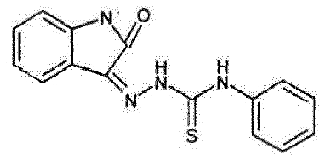 ist.

14. Verbindung nach Anspruch 13, wobei R¹⁴ Fluor oder Halogenalkyl, insbesondere Fluoralkyl ist; insbesondere wobei R¹⁴ Fluor oder Fluoralkyl ist; X CH ist
und R¹, R² und R³ jeweils H sind; ganz besonders wobei die Verbindung die folgende Formel aufweist: 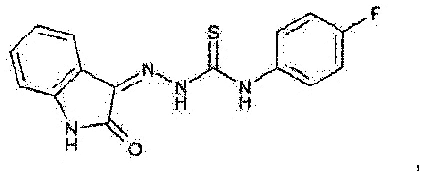 oder wobei die Verbindung die folgende Formel aufweist:

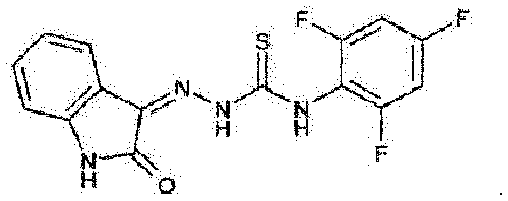

15. Zusammensetzung, die eine Menge der Verbindung nach einem der Ansprüche 12 bis 14, die zum Hemmen des Wachstums von neoplastischen Zellen in einer Person wirksam ist, und einen pharmazeutisch unbedenklichen Trägerstoff umfasst; insbesondere wobei die Zusammensetzung weiterhin eine wirksame Menge einer antineoplastischen Substanz umfasst, die aus den Mikrotubuli bindenden Mitteln, DNA-Interkalatoren, DNA-Alkylierungsmitteln, DNA-Vernetzern, DNA-Synthesehemmern, DNA- und/oder RNA-Transkriptionshemmern, Enzymhemmern, Genregulatoren, Enzymen, Antikörpern und Angiogenesehemmern ausgewählt ist.

16. Verbindung zur Verwendung nach den Ansprüchen 2, 5 bis 6, 8 oder 9, wobei das tiefgestellte a 1 ist.

17. Verbindung zur Verwendung nach den Ansprüchen 2, 5 bis 6, 8 oder 9, wobei das tiefgestellte a 2 bis 5 ist.

18. Verbindung nach Anspruch 13 oder 14, wobei das tiefgestellte a 1 ist.

19. Verbindung nach Anspruch 13 oder 14, wobei das tiefgestellte a 2 bis 5 ist.

## Revendications

1. Un composé possédant la formule suivante : 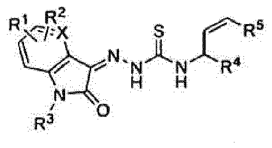 où R¹ et R² indépendamment sont sélectionnés parmi H, fluoro et iodo ; X est CH ;
R³ est H ;
R⁴ est H ; ou, avec R⁵, forme un cycle naphtyle ou phényle optionnellement substitué ;
R⁵ est H ; ou, avec R⁴ forme un cycle naphtyle ou phényle optionnellement substitué ;
et
lorsque R⁴ et R⁵ forment un fragment para-méthoxyphényl, au moins R¹ ou R² est autre que H ;
à condition que le composé ne soit pas un des composés suivants : 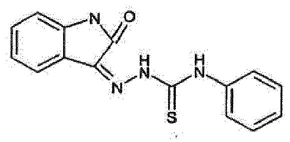 ou 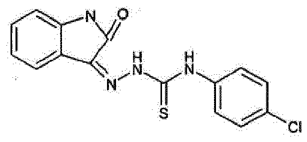 pour l'utilisation dans un procédé d'inhibition de la croissance des cellules résistantes aux médicaments chez un sujet, consistant à identifier un sujet ayant des cellules résistantes aux médicaments et à administrer le composé au sujet.

2. Le composé destiné à l'utilisation décrite à la revendication 1, le composé possédant la formule suivante : 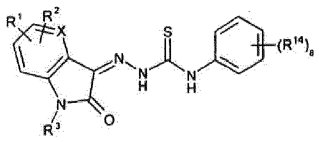 où a est 0 à 5;
R¹⁴ est un halogène ; -OR¹⁵ ; -NR¹⁶R¹⁷ ; un cyano ; un nitro ; un haloalkyle ; un alkyle inférieur ; ou un carboxy, et chaque R¹⁴ peut être identique ou différent ;
R¹⁵ est un alkyle inférieur ;
R¹⁶ est un alkyle inférieur ; et
R¹⁷ est un alkyle inférieur ;
où l'alkyle inférieur est un méthyle, un éthyle, un *n*-propyle, un isopropyle, un *n*-butyle, un isobutyle, un *t*-butyle, un pentyle, un hexyle, un heptyle, un octyle ou un décyle.

3. Le composé destiné à l'utilisation décrite à la revendication 1, le composé possédant la formule suivante : 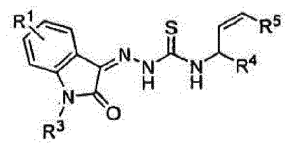 où R¹, R³, R⁴ et R⁵ sont comme indiqué ci-dessus ; ou où le composé possède la formule suivante :

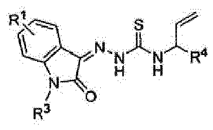

4. Le composé destiné à l'utilisation décrite à la revendication 1, où le composé possède la formule suivante : 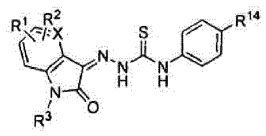 où R¹⁴ est H ; un halogène ; -OR¹⁵ ; -NR¹⁶R¹⁷ ; un cyano ; un nitro ; un haloalkyle ; un alkyle inférieur ; ou un carboxy ; ou où le composé possède la formule suivante : 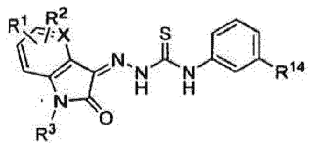 où R¹⁴ est H ; un halogène ; -OR¹⁵ ; -NR¹⁶R¹⁷ ; un cyano ; un nitro ; un haloalkyle ; un alkyle inférieur ; ou un carboxy ; ou où le composé possède la formule suivante : 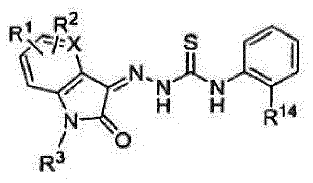 où R¹⁴ est H ; un halogène ; -OR¹⁵ ; -NR¹⁶R¹⁷ ; un cyano ; un nitro ; un haloalkyle ; un alkyle inférieur ; ou un carboxy ;
où l'alkyle inférieur est un méthyle, un éthyle, un n-propyle, un isopropyle, un *n*-butyle, un isobutyle, un *t*-butyle, un pentyle, un hexyle, un heptyle, un octyle ou un décyle.

5. Le composé destiné à l'utilisation décrite aux revendications 2 à 4, où R¹⁴ est -OMe ; ou où R¹⁴ est un haloalkyle.

6. Le composé destiné à l'utilisation décrite à la revendication 2, où R¹⁴ est un fluoro ou un fluoroalkyle.

7. Le composé destiné à l'utilisation décrite à la revendication 1, où le composé possède la formule suivante : 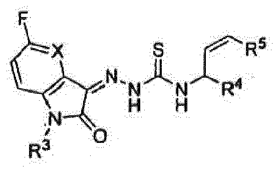 ou où le composé possède la formule suivante : 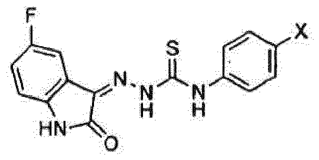 où X est sélectionné parmi H, -OCH₃, -CH₃, -CH₂CH₃, -F, -Cl, -Br, -I, - CF₃, -OCF₃, -NO₂, un phényle, -N₃, -CN, -OH, -NH₂, -NMe₂, -COOH et - SO₃ ; ou où le composé possède la formule suivante : 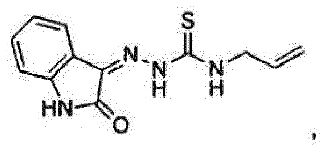 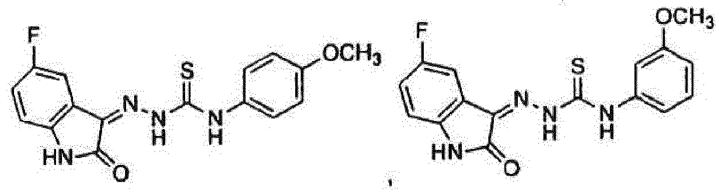 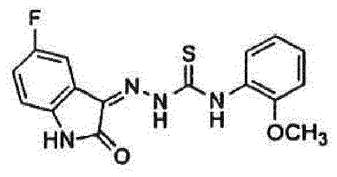 ou où le composé possède la formule suivante : 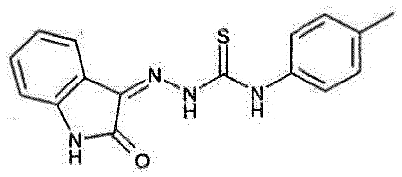 ou où le composé possède la formule suivante : 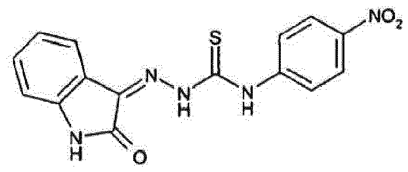 ou où le composé possède la formule suivante : 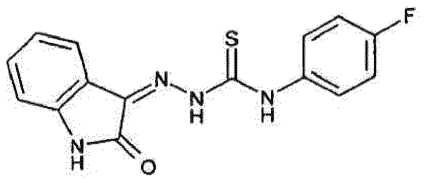 ou où le composé possède la formule suivante :

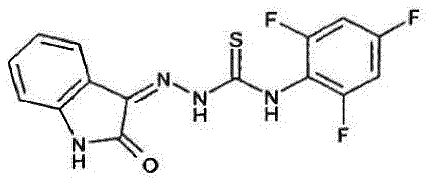

8. Le composé destiné à l'utilisation décrite à n'importe laquelle des revendications 1 à 7, dans lequel les cellules présentent un phénotype de résistance multimédicamenteuse ; ou supposant également de resensibiliser les cellules à un substrat MDR1 ; ou dans lequel les cellules sont des cellules néoplastiques.

9. Le composé destiné à l'utilisation décrite aux revendications 1 à 8, dans lequel les cellules comprennent le cancer ; en particulier, dans lequel le cancer comprend le cancer du cerveau, le cancer du sein, le cancer de la vessie, le cancer des os, le cancer du col de l'utérus, le cancer du colon, le cancer du système nerveux central, le cancer de l'oesophage, le cancer de la vésicule biliaire, le cancer gastro-intestinal, le cancer de la tête et du cou, la maladie de Hodgkin, les lymphomes non hodgkiniens, le cancer laryngien, la leucémie, le cancer des poumons, le mélanome, le neuroblastome, le cancer des ovaires, le cancer du pancréas, le cancer de la prostate, le cancer rectal, le cancer rénal, le rétinoblastome, le cancer de l'estomac, le cancer des testicules, la tumeur de Wilms ou une combinaison de ces maladies.

10. Le composé destiné à l'utilisation décrite à la revendication 1, dans lequel le procédé suppose d'inhiber le développement de la résistance multimédicamenteuse dans les troubles hyperproliférants ; ou dans lequel le sujet a déjà été soigné avec au moins un substrat MDR1.

11. Le composé destiné à l'utilisation décrite à n'importe laquelle des revendications 1 à 10, dans lequel le composé est administré en combinaison avec un agent cytotoxique ; en particulier, dans lequel l'agent cytotoxique est un agent anticancer ; plus particulièrement, dans lequel l'agent anticancer est sélectionné parmi les agents de liaison microtubulaires, les intercalateurs d'ADN, les agents d'alkylation d'ADN, les agents de réticulation d'ADN, les inhibiteurs de synthèse d'ADN, les inhibiteurs de transcription d'ADN et/ou d'ARN, les inhibiteurs d'enzyme, les régulateurs de gènes, les enzymes, les anticorps et les inhibiteurs de l'angiogénèse; plus particulièrement encore, dans lequel l'agent anticancer est sélectionné parmi les éléments suivants : erlotinib, gefitinib, temozolomide, paclitaxel, docetaxel, daunorubicine, cisplatine, carboplatine, oxaliplatine, colchicine, dolastatine 15, nocodazole podophyllotoxine, rhizoxine, vinblastine, vindésine, vinorelbine (navelbine), les épothilones, les mitomycines, bléomycine, chlorambucile, carmustine, melphalan, mitoxantrone, 5-fluoro-5'-déoxyuridine, camptothécine, SFTI-1, topotécan, irinotécanétoposide, ténoposide, geldanamycine, méthotrexate, adriamycine, actinomycine D, médroxyprogestérone, mifepristone, raloxifène, 5-azacytidine, 5-aza-2'-déoxycytidine, zébularine, tamoxifène, 4-hydroxytamoxifène, apigénine, rapamycine, angiostatine K1-3, L-asparaginase, staurosporine, génistéine, fumagilline, endostarine, ypérite d'isophosphoramide, thalidomide et leurs analogues.

12. Une composition contenant une quantité du composé de n'importe laquelle des revendications 1 à 11 capable d'inhiber la croissance des cellules néoplastiques chez un sujet et un vecteur pharmaceutiquement acceptable destiné à l'utilisation dans un procédé d'inhibition de la croissance des cellules résistantes aux médicaments chez le sujet, consistant à identifier un sujet ayant des cellules résistantes aux médicaments et à lui administrer le composé ; en particulier où la composition comporte également une quantité efficace d'un agent anticancer sélectionné parmi les agents de liaison microtubulaires, les intercalateurs d'ADN, les agents d'alkylation d'ADN, les agents de réticulation d'ADN, les inhibiteurs de synthèse d'ADN, les inhibiteurs de transcription d'ADN et/ou d'ARN, les inhibiteurs d'enzyme, les régulateurs de gènes, les enzymes, les anticorps et les inhibiteurs de l'angiogénèse.

13. Un composé de la formule suivante : 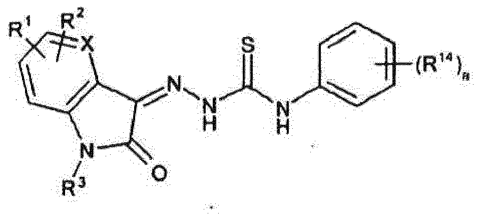 où a est 0 à 5;
où R¹ et R² indépendamment sont sélectionnés parmi H ; et iodo ;
X est CH ;
R³ est H ;
et
R¹⁴ est un fluoro ; un iodo ; -OR¹⁵ ; -NR¹⁶R¹⁷ ; un cyano ; un haloalkyle ; un carboxy ; un éthyle, un *n*-propyle, un isopropyle, un *n*-butyle, un isobutyle, un *t*-butyle, un pentyle, un hexyle, un heptyle, un octyle ou un décyle ; et chaque R¹⁴ peut être identique ou différent ;
R¹⁵ est un éthyle, un *n*-propyle, un isopropyle, un *n*-butyle, un isobutyle, un *t*-butyle, un pentyle, un hexyle, un heptyle, un octyle ou un décyle ;
R¹⁶ est un alkyle inférieur ; et
R¹⁷ est un alkyle inférieur ;
où l'alkyle inférieur est un méthyle, un éthyle, un *n*-propyle, un isopropyle, un *n*-butyle, un isobutyle, un *t*-butyle, un pentyle, un hexyle, un heptyle, un octyle ou un décyle ; ou un composé possédant la formule suivante : 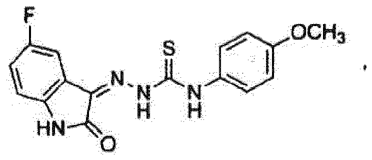 à condition que le composé ne soit pas :

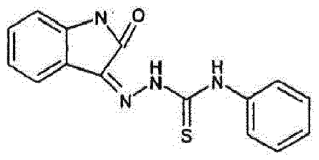

14. Le composé de la revendication 13 où R¹⁴ est un fluoro ou un haloalkyle, en particulier un fluoroalkyle ; en particulier, où R¹⁴ est un fluoro ou un fluoroalkyle ; X est CH ;
et R¹, R² et R³ sont chacun H ; plus particulièrement, où le composé possède la formule suivante : 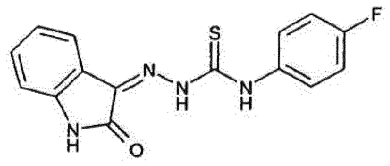 ou où le composé possède la formule suivante :

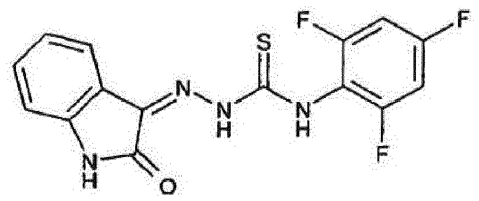

15. Une composition contenant une quantité du composé de n'importe laquelle des revendications 12 à 14 capable d'inhiber la croissance des cellules néoplastiques chez un sujet et un vecteur pharmaceutiquement acceptable ; en particulier où la composition comporte également une quantité efficace d'un agent anticancer sélectionné parmi les agents de liaison microtubulaires, les intercalateurs d'ADN, les agents d'alkylation d'ADN, les agents de réticulation d'ADN, les inhibiteurs de synthèse d'ADN, les inhibiteurs de transcription d'ADN et/ou d'ARN, les inhibiteurs d'enzyme, les régulateurs de gènes, les enzymes, les anticorps et les inhibiteurs de l'angiogénèse.

16. Le composé destiné à l'utilisation décrite aux revendications 2, 5 à 6, 8 ou 9, où l'indice inférieur a est 1.

17. Le composé destiné à l'utilisation décrite aux revendications 2, 5 à 6, 8 ou 9, où l'indice inférieur a est 2 à 5.

18. Le composé des revendications 13 ou 14, où l'indice inférieur a est 1.

19. Le composé des revendications 13 ou 14, où l'indice inférieur a est 2 à 5.
